# EUROPEAN PATENT SPECIFICATION

(11) **EP 1 715 046 B1**
(45) Date of publication and mention of the grant of the patent: **29.04.2009**
(21) Application number: 06012377.5
(22) Date of filing: 31.03.1994
(51) Int. Cl.: C12N 15/31, C12N 15/62, C12P 21/02, A61K 39/02, C12R 1/92

(54) **New polypeptide, DNA encoding the polypeptide, recombinant vector bearing the DNA and recombinant virus utilizing the recombinant vector as well as use thereof**
Polypeptid, dafür kodierende DNA, diese DNA beinhaltender, rekombinanter Vektor, Herstellung eines rekombinanten Virus unter Verwendung dieses Vektors und dessen Anwendung
Nouveau polypeptide, ADN codant pour ledit polypeptide, vecteur de recombinaison contenant ledit ADN, virus de recombinaison préparé en utilisant ledit vecteur et son utilistation

(30) Priority: 31.03.1993 JP 7413993; 30.09.1993 JP 24562593
(43) Date of publication of application: 25.10.2006
(62) Divisional of application: 04013109.6
(73) Proprietor: Zeon Corporation, Tokyo 100-8246 (JP)
(72) Inventor: Saito, Shuji, c/o Zeon Corporation, Tokyo 100-8246 (JP); Ohkawa, Setsuko, Yokohama-shi, Kanagawa 222-0026 (JP); Saeki, Sakiko, c/o Zeon Corporation, Tokyo 100-8246 (JP); Ohsawa, Ikuroh, Tokyo 161 (JP); Funato, Hirono, Soka-shi, Saitama 340 (JP); Iritani, Yoshikazu, c/o Shionogi & Co.,Ltd., Osaka 541-0045 (JP); Aoyama, Shigemi, Koka-gun, Shiga 528 (JP); Takahashi, Kiyohito, Kurita-gun, Shiga 520-30 (JP)
(74) Representative: Bufton, Karen A.J.

(56) References cited:
- EP-A- 0 308 220
- EP-A- 0 345 021
- EP-A- 0 404 576
- EP-A- 0 603 406
- US-A- 5 196 514
- AVAKIAN, A.P., ET AL .: "EVALUATION OF SODIUM DODECYL SULFATE-POLYACRYLAMIDE GEL ELECTROPHORESIS PURIFIED PROTEINS OF MYCOPLASMA GALLISEPTICUM AND M. SYNOVIAE AS ANTIGENS IN A DOT-ENZYME-LINKED IMMUNOSORBENT ASSAY" AVIAN DISEASES, vol. 34, 1990, pages 575-584, XP000609379
- BARBOUR, E.K., ET AL .: "IDENTIFICATION OF THE ANTIGENIC COMPONENTS OF THE VIRULENT MYCOPLASMA GALLISEPTICUM (R) IN CHICKEN: THEIR ROLE IN DIFFERENTIATION FROM THE VACCINE STRAIN (F)" VETERINARY IMMUNOLOGY AND IMMUNOPATHOLOGY, vol. 21, 1989, pages 197-206, XP000608919
- HORST J ET AL: "ON PROCARYOTIC GENE EXPRESSION IN EUCARYOTIC SYSTEMS" HUMAN GENETICS, BERLIN, DE, vol. 54, no. 3, 1980, pages 289-302, XP001078797 ISSN: 0340-6717

## Description

### TECHNICAL FIELD

The present invention relates to a novel polypeptide showing antigenicity to Mycoplasma gallisepticum, a fused polypeptide between the said polypeptide and a signal membrane anchor, and a recombinant Avipox virus capable of expressing a polypeptide showing antigenicity to Mycoplasma gallisepticum, especially a polypeptide showing antigenicity on the membrane surface of a host cell, as well as use thereof.

### BACKGROUND

It is expected that a polypeptide showing antigenicity to Mycoplasma gallisepticum can be utilized as an effective ingredient of a vaccine for Mycoplasma gallisepticum infections, since an egg-laying rate and a hatching rate of eggs produced by infected chickens are markedly reduced when infected with Mycoplasma gallisepticum. At present, the system using Escherichia coli or yeast is known to prepare the antigenic protein of Mycoplasma gallisepticum by genetic engineering (Japanese Patent Application Laid-Open No. 2-111795). In general, it is pointed out that the production of a polypeptide in the system using bacteria involves problems that firstly an antigen is expressed in a less amount and secondly, a pyrogen originating in a host cannot be removed. It is thus the actual situation that such a system has not been practically applied yet. For this reason, studies have been made on the preparation of a polypeptide expressing an antigenicity or a recombinant live vaccine, using a recombinant virus. However, as far as Mycoplasma gallisepticum is concerned, any recombinant virus inserted with DNA encoding said protein has not been prepared.

In a virus protein where the virus infects cells, one type of a protein expressed is transported to the cell surface and the protein is expressed on the surface of a cell membrane (hereinafter such a state is sometimes merely referred to as being expressed on the cell surface) and another type of a protein that is not expressed on the cell surface. A representative example of the former protein is a glycoprotein contained in the coat of a virus. A recombinant virus that expresses such a protein efficiently exhibits the protein on the cell surface. It is thus considered that a high antibody titer can be induced in poultry infected with this recombinant virus (Japanese Patent Application Laid-Open No. 1-157381). On the other hand, an example of the latter type of protein includes a protein originating in bacteria, such as an antigenic protein of Mycoplasma gallisepticum.

It is not expectable to induce a high antibody titer from such recombinant viruses that express these proteins, since they are expressed on the cell membrane surface merely in an extremely small quantity. However, if such a protein can be expressed on the cell membrane surface in a large quantity by genetic engineering, a high antibody titer will be induced. Thus, investigations have been made to express on the membrane surface such a protein that is not principally expressed on the membrane surface. For example, there is a report that DNA encoding a signal protein having the function of secreting a protein on the cell membrane surface and DNA encoding a membrane anchor protein having the function of retaining the secreted protein so as not to leave out of the cell membrane surface are ligated with the 5' end and the 3' end of DNA encoding an antigenic protein, respectively, and a recombinant vaccinia virus inserted with the resulting hybrid DNA expresses the antigenic protein on the cell membrane surface of a host (J. Viol., 64, 4776-4783 (1990) or Mol. Cell. Biol., 6, 3191-3199 (1986)). However, DNA encoding a signal and DNA encoding a membrane anchor are independently ligated with DNA encoding an antigenic protein in these examples so that it is hardly applicable practically due to complicated preparation of a recombinant virus.

### DISCLOSURE OF THE INVENTION

The present inventors have made extensive studies to provide a polypeptide having antigenicity originating in Mycoplasma and showing a high antigenicity, a polypeptide having antigenicity to Mycoplasma gallisepticum expressed on the cell membrane surface especially in a large quantity, DNA encoding the polypeptide, a recombinant virus inserted with the same DNA and a vaccine utilizing the virus. As a result, the present invention has come to be accomplished.

In a first aspect, the invention provides a substantially pure antigenic protein which is reactive with Mycoplasma gallisepticum-immunized sera or Mycoplasma gallisepticum-infected sera and which has the amino acid sequence shown by SEQ ID NO: 27.

The invention further provides an isolated DNA encoding the antigenic protein.

The invention also provides a fused protein comprising a polypeptide of Mycoplasma gallisepticum ligated at the N-terminus thereof with a signal membrane anchor of type II external membrane protein of a virus that infects poultry, wherein said polypeptide of Mycoplasma gallisepticum has the amino acid sequence shown by SEQ ID NO: 27.

The signal membrane anchor may be encoded by DNA comprising SEQ ID NO: 13. A hydrophilic sequence having between 10 and 50 amino acids may be present at the carboxy terminal of the signal membrane anchor.

Also provided is a hybrid DNA encoding the fused protein.

In a further aspect, the invention provides a component vaccine comprising a protein according to the invention.

In a further aspect, the invention provides a recombinant Avipox virus comprising DNA encoding a polypeptide of Mycoplasma gallisepticum, wherein said polypeptide of Mycoplasma gallisepticum has the amino acid sequence shown by SEQ ID NO: 27.

The polypeptide of Mycoplasma gallisepticum may have at the N-terminus thereof, a signal membrane anchor of a type II external membrane protein from a virus that infects poultry.

Further provided is a live vaccine for poultry Mycoplasma gallisepticum infection comprising a live recombinant Avipox virus according to the invention.

### BRIEF DESCRIPTION OF THE DRAWINGS

Fig. 1 shows a restriction enzyme map of DNA including the open reading frame of TM-81.
Fig. 2 shows the procedure for construction of TTM-1N and TTM-1C.
Fig. 3 shows the procedure for constructing pNZ7929-R1.
Fig. 4 shows the procedure for constructing pNZ87N.
Fig. 5 shows the procedure for constructing pNZ7929-R2.
Fig. 6 (A) and (B) show the procedure for constructing pNZ2929XM1.
Fig. 7 shows a restriction enzyme map of DNA including the open reading frame of TTM-1 polypeptide.
Fig. 8 shows a restriction enzyme map of DNA including the open reading frame of TM-67 polypeptide and the position of synthetic primers on ORF.
Fig. 9 (A) and (B) show the procedure for constructing pHZ79Z9-67.
Fig. 10 shows a restriction enzyme map of DNA including the open reading frame of TM-66 polypeptide and the position of synthetic primers on ORF.
Fig. 11(A), 11(B) and 11(C) show the procedure for constructing pTM66.
Fig. 12 shows the procedure for constricting pNZ7929-66.
Fig. 13 shows a restriction enzyme map of DNA encoding the full length of TM-16 polypeptide.
Fig. 14 shows a restriction enzyme map of the open reading frame of TM-16 polypeptide.

### BEST MODE FOR PRACTICING THE INVENTION

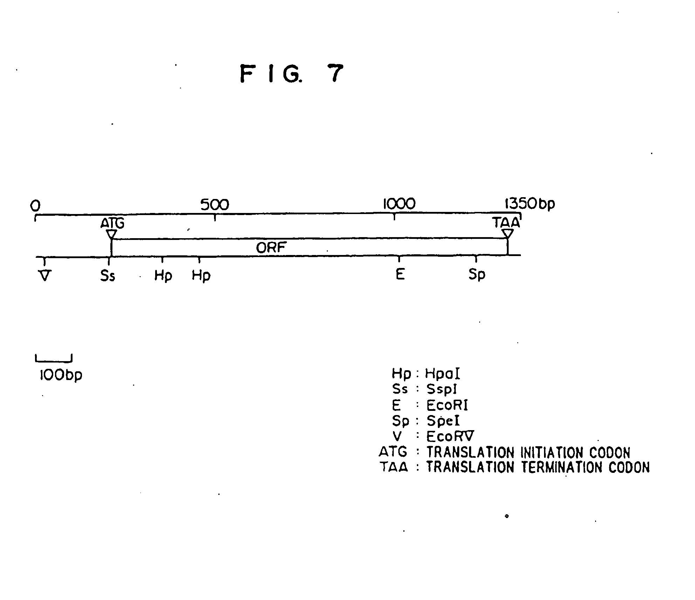

Described herein is a novel polypeptide which shows an antigenicity which originates in Mycoplasma gallisepticum having a high antigenicity, and which includes a polypeptide showing an antigenicity which causes an antigen-antibody reaction with sera immunized with Mycoplasma gallisepticum or sera and which is encoded by the DNA sequence having the restriction enzyme map shown in Fig. 7 originating in Mycoplasma gallisepticum, or a modified polypeptide thereof. Specific examples of the polypeptide having such an polypeptide include those showing an antigenicity and having amino acid sequences of SEQ ID NOS: 1, 15, 16 and 27. The modified polypeptide showing an antigenicity referred to herein is a polypeptide in which the amino acid sequence is modified by substitution, loss, deletion, insertion or addition but which shows an antigenicity comparable to that of the aforesaid polypeptide. Taking SEQ ID NO: 1 as an example, a modified polypeptide is used to mean a polypeptide having the same antigenicity as in an antigenic protein having the amino acid sequence equivalent thereto and having a homology of at least 70% to the amino acid sequence of said polypeptide, preferably 80% or more, most preferably 90% or more. The homology referred to herein is used to mean the homology determined as an index by DNA sequencing input analysis system "DNASIS" (marketed by Takara Shuzo Co.).

Hereinafter a sequence number is sometimes simply referred to as sequence in the specification. For example, Sequence No. 1 is sometimes referred to as Sequence 1.

Furthermore, the DNA which encodes the polypeptide showing an antigenicity described herein includes DNA encoding a polypeptide in which the amino acid is modified by deletion, additions, insertion, loss, substitution, etc., so long as it causes an antigen-antibody reaction with sera immunized with Mycoplasma gallisepticum or sera and shows an antigenicity originating in Mycoplasma gallisepticum or an antigenicity equivalent thereto.

Avipox virus which is a second aspect of the present invention is a recombinant Avipox virus inserted with a hybrid DNA in which DNA encoding the antigenic polypeptide of Mycoplasma gallisepticum (hereinafter abbreviated as antigenic DNA) or DNA encoding a signal membrane anchor of Type II external membrane protein is ligated with DNA encoding a polypeptide showing an antigenicity of Mycoplasma gallisepticum. In order to express large quantities of the polypeptide showing an antigenicity of Mycoplasma gallisepticum that is not basically expressed on the surface of cell membrane, it is preferred to employ the hybrid DNA.

That is, in the second aspect of the present invention, there are provided a polypeptide showing an antigenicity of Mycoplasma gallisepticum (hereinafter sometimes merely referred to as antigenic protein), a fused polypeptide ligated at the N terminus of the polypeptide with a signal membrane anchor of type II outer membrane protein of a virus infected to poultry (hereinafter merely referred to as signal membrane anchor), a vaccine against Mycoplasma gallisepticum infections comprising as an effective ingredient the antigenic protein or the fused polypeptide, a hybrid DNA which encodes the fused polypeptide, a recombinant Avipox virus inserted into the genomic region non-essential to growth of Avipox virus (hereinafter referred to as non-essential region) with DNA encoding the antigenic protein or the hybrid DNA, and a live vaccine against Mycoplasma gallisepticum which comprises the Avipox virus as an effective ingredient.

The signal membrane anchor which is employed in the present invention as the second aspect is a polypeptide region having the function of transporting type II external membrane protein of a virus infected to poultry to the surface of cell membrane and expressing the transported protein on the surface of cell membrane, and is preferably derived from a virus which is non-pathogenic to human. The DNA encoding the signal membrane anchor which is employed in the present invention (hereinafter referred to as signal membrane anchor DNA) can be readily found by amino acid sequencing analysis of the hydrophobic peptide region of type II external membrane protein at the amino terminus. A specific example of the signal membrane anchor is that having the sequence shown by SEQ ID NO: 13 (Mol. Cell. Biol., 10, 449-457 (1990)). This DNA codes for 22 amino acids at the amino terminus of hemagglutinin neuraminidase (hereinafter abbreviated as HN protein) of Newcastle disease virus (hereinafter abbreviated as NDV).

In order to stably exhibit the expressed antigenic protein on the cell membrane, it is effective for a hydrophilic peptide to be present at the carboxy terminal of the signal membrane anchor. Accordingly, it is preferred that DNA encoding a hydrophilic peptide be added downstream the signal membrane anchor DNA. DNA to be added comprises base pairs corresponding to 10 to 50 amino acids, preferably 20 to 30 amino acids.

Specific examples of the DNA encoding the antigenic protein described herein, in addition to the four sequences described herein, DNA described in Japanese Patent Application Laid-Open No. 1-111795, a genomic DNA fragment of Mycoplasma gallisepticum containing the aforementioned DNA, DNA (hereinafter referred to as TTM-1) encoding a polypeptide of about 40 kilodaltons showing an antigenicity and having the sequence shown by SEQ. ID NO: 14 (hereinafter referred to as TTM-1' polypeptide), DNA derived from natural Mycoplasma gallisepticum substantially equivalent to TTM-1' (hereinafter referred to as TTM-1), and the like. The TTM-1 and 1' are disclosed in WO 93/24646. The DNA encoding the antigenic protein may also be DNA encoding such a polypeptide that a part of the sequence is modified by substitution, loss, deletion, insertion, addition, etc. as long as it retains an antigenicity substantially equivalent to that of the antigenic protein encoded by the nucleotide sequence.

Sources for collecting such a DNA may be any of the sources so long as they belong to Mycoplasma gallisepticum. Specific examples include S6 strain (ATCC 15302), PG31 (ATCC 19610) and the like.

The hybrid DNA which is used in the present invention as its second aspect is the aforesaid signal membrane anchor DNA ligated with DNA encoding a polypeptide showing an antigenicity. The fused polypeptide of the present invention is a polypeptide encoded by the hybrid DNA described above which contains a part of the signal membrane anchor and a part of the polypeptide showing an antigenicity in the molecule of the polypeptide. The hybrid DNA can be produced in a conventional manner, e.g., by modifying the 3' end of the signal membrane anchor DNA and the 5' end of the DNA encoding the antigenic protein so as to form ligatable restriction enzyme digestion fragments, and ligating both DNAs according to the method for ligation using a ligase or the method for ligating both DNAs with a ligase by inserting an appropriate linker therebetween. The signal membrane anchor and the DNA encoding the polypeptide showing an antigenicity may contain therebetween, for example, DNA encoding a hydrophilic peptide, DNA encoding other antigenic protein, linker DNA, etc., so long as the signal membrane anchor DNA and the DNA encoding the polypeptide showing an antigenicity are expressed as one polypeptide. The fused polypeptide of the present invention is obtained by incubating a recombinant Avipox virus, later described, in culture cells such as chick embryo fibroblast (hereinafter referred to as CEF cells) or embryonated chorioallantoic membrane cells, etc., and purifying the desired polypeptide by a method optionally chosen from chromatography, precipitation by salting-out, density gradient centrifugation, etc. The fused polypeptide thus obtained can be used as a component vaccine which will be later described.

The recombinant Avipox virus of the present invention is a recombinant Avipox virus in which the aforesaid DNA or hybrid DNA is inserted in the non-essential region. The recombinant Avipox virus of the present invention may be constructed in a conventional manner, e.g., by the method described in Japanese Patent Application Laid-Open No. 1-168279. That is, the non-essential region of Avipox virus is incorporated into a DNA fragment, if necessary, inserted with a promoter in the non-essential region, to construct a first recombinant vector.

As the non-essential region of Avipox virus which is used in the present invention, there are a TK gene region of quail pox virus, a TK gene region of turkey pox virus and DNA fragments described in Japanese Patent Application Laid-Open 1-168279, preferably a region which causes homologous recombination with EcoRI fragment of about 7.3 Kbp, HindIII fragment of about 5.2 Kbp, EcoRI-HindIII fragment of about 5.0 Kbp, BamHI fragment of about 4.0 Kbp, described in the patent specification supra.

Examples of the vector used in the present invention include plasmids such as pBR322, pBR325, pBR327, pBR328, pUC7, pUC8, pUC9, pUC19, and the like; phages such as λ phage, M13 phage, etc.; cosmid such as pHC79 (Gene, 11, 291, 1980) and the like.

The Avipox virus used in the present invention is not particularly limited so long as it is a virus infected to poultry. Specific examples of such a virus include pigeon pox virus, fowl pox virus (thereafter abbreviated as FPV), canary pox virus, turkey pox virus, preferably turkey pox virus, pigeon pox virus and FPV, more preferably pigeon pox virus and FPV. Specific examples of the most preferred Avipox virus include FPVs such as ATCC VR-251, ATCC VR-249, ATCC VR-250, ATCC VR-229, ATCC VR-288, Nishigahara strain, Shisui strain, CEVA strain and a viral strain among CEVA strain-derived viruses which forms a large plaque when infected to chick embryo fibroblast, and a virus such as NP strain (chick embryo-conditioned pigeon pox virus Nakano strain), etc. which is akin to FPV and used as a fowlpox live vaccine strain. These strains are commercially available and readily accessible.

Then, the aforesaid antigenic DNA or hybrid DNA is inserted into the non-essential region of the first recombinant vector described above to construct a second recombinant vector. Where the hybrid DNA is employed, a promoter is generally inserted upstream the hybrid DNA. The promoter used may be a promoter having any nucleotide sequence, irrespective of a synthetic or natural promoter, as far as it effectively functions as a promoter in the system of transcription possessed by APV. Accordingly, not only a promoter inherent to APV such as a promoter of APV gene encoding thymidine kinase but also DNA derived from viruses other than APV and DNA derived from eucaryote or procaryote may also be employed in the present invention, as long as these substances meet the requirements described above. Specific examples of such a promoter include a promoter of vaccinia virus (hereinafter sometimes abbreviated as VV) described in J. Virol., 51, 662-669 (1984), more specifically a promoter of VV DNA encoding 7.5 K polypeptide, a promoter of VV DNA encoding 19 K polypeptide, a promoter of VV DNA encoding 42 K polypeptide, a promoter of VV DNA encoding thymidine kinase, a promoter of VV DNA encoding 28 K polypeptide, etc. Furthermore, there may be used a synthetic promoter obtained by modification of the Moss et al. article (J. Mol. Biol., 210, 749-776, 771-784, 1989), a promoter synthesized by Davidson, a promoter obtained by modifying a part of the Davidson promoter through deletion or change within such a range that does not lose the promoter activity (e.g., T T T T T T T T T T T T G G C A T A T A A A T A A T A A T A A A T A C A A T A A T T A A T T A C G C G T A A A A A T T G A A A A A C T A T T C T A A T T T A T T G C A C T C or T T T T T T T T T T T T T T T T T T T T G C C A T A T A A A T A A T A A A T A C A A T A A T T A A TTACGCGTAAAAATTGAAAAACTAT T C T A A T T T A T T G C A C T C etc.).

Further in view of easy detection of the recombinant virus, a marker DNA such as DNA encoding β-galactosidase may also be inserted.

The recombinant Avipox virus may be constructed by transfecting the second recombinant vector described above to animal culture cells previously infected with Avipox virus and causing homologous recombination between the vector DNA and the viral genome DNA. The animal culture cells used herein may be any cells in which Avipox can grow. Specific examples of such animal culture cells are CEF cells, embryonated egg chorioallantoic membrane cells, and the like.

The desired recombinant Avipox virus is isolated from the virus infected to host cells by the method of plaque hybridization, etc. The recombinant Avipox virus may be further purified by plaque assay, etc.

The recombinant virus of the present invention constructed by the method described above can be inoculated to fowl as a live vaccine against Mycoplasma gallisepticum infection.

The live vaccine of the present invention is prepared by, e.g., the following method, though the process is not particularly limited. The recombinant virus of the present invention is infected to cells in which the virus can grow (hereafter referred to as host cells). After the recombinant virus grows, the cells are recovered and homogenated. The homogenate is centrifuged with a centrifuging machine to separate into the precipitates and the high titer supernatant containing the recombinant virus in a centrifuging tube. The resulting supernatant is substantially free of host cells but contains the cell culture medium and the recombinant virus and hence can be used as a live vaccine. The supernatant may be diluted by adding a pharmacologically inactive carrier, e.g., physiological saline, etc. The supernatant may be freeze-dried to be provided for use as a live vaccine. A method for administration of the live vaccine of the present invention is not particularly limited and examples of the administration include a method for scratching the skin and inoculating the live vaccine on the scratch, effecting the inoculation through injection, oral administration by mixing the live vaccine with feed or drinking water, inhalation by aerosol or spray, etc. In order to use as the live vaccine, the dosage may be the same as ordinary live vaccine; for example, approximately 10² to 108 plaque forming unit (hereinafter abbreviated as PFU) is inoculated per chick. Where the inoculation is effected by injection, the recombinant virus of the present invention is generally suspended in about 0.1 ml of an isotonic solvent such as physiological saline and the resulting suspension is provided for use. The live vaccine of the present invention may be stored under ordinary conditions and provided for use. For example, when the recombinant virus of the present invention is freeze-dried, it is possible to store at room temperature (20 to 22°C). It is also possible to freeze the virus suspension at -20 to -70°C and store the frozen suspension.

On the other hand, the component vaccine of the present invention comprises as an effective ingredient the polypeptide showing an antigenicity in accordance with the present invention, especially the fused polypeptide. The component vaccine may be administered to fowl in the same manner as in the live vaccine described above. The dose is generally in the range of approximately 1 µg to 1 mg per one subject.

According to the present invention, the polypeptide showing an Mycoplasma gallisepticum antigenicity and the, fused polypeptide between the said polypeptide and the signal membrane anchor are obtained. In particular, this fused polypeptide is effective as a vaccine against Mycoplasma gallisepticum infections. By utilizing DNA encoding the fused protein, the recombinant Avipox virus which can express the polypeptide showing an Mycoplasma gallisepticum antigenicity is obtained. The recombinant Avipox virus is effective as a potent live vaccine against Mycoplasma gallisepticum infections. In addition, the novel polypeptide showing an antigenicity of the present invention and DNA encoding the same can be utilized as a component vaccine and a live vaccine, respectively.

### EXAMPLES

Hereinafter the present invention will be described with reference to the examples and the reference examples but is not deemed to be limited thereto.

### Reference Example 1

Obtaining of polypeptide DNA TTM-1 in which Mycoplasma gallisepticum is expressed:

### (1) Preparation of genomic DNA of Mycoplasma gallisepticum

Mycoplasma gallisepticum S6 strain was cultured at 37°C for 3 to 5 days in liquid medium prepared by supplementing 20% horse serum, 5% yeast extract, 1% glucose and a trace amount of phenol red as a pH indicator in 100 ml of PPLO broth basal medium. As Mycoplasma gallisepticum proliferated, pH of the culture broth decreased. At the point of time when the color of the pH indicator contained in the culture broth changed from red to yellow, incubation was terminated. The culture medium was centrifuged at 8000G for 20 minutes to collect the cells. The cells were then suspended in 1/10 volume of PBS based on the volume of culture medium. The suspension was again centrifuged at 10,000 rpm x G for 20 minutes to collect the cells. The collected cells were resuspended in 2.7 ml of PBS and SDS was added thereto in a final concentration of 1%. Furthermore 10 µg of RNase was added to the mixture. The mixture was incubated at 37°C for 30 minutes to cause lysis.

The lysate was extracted 3 times with an equal volume of phenol and then 3 times with ethyl ether. The extract was precipitated with ethanol to give 200 µg of genomic DNA of Mycoplasma gallisepticum.

### (2) Genomic Southern hybridization of Mycoplasma gallisepticum using TM-1 DNA as a probe

After 1 µg of Mycoplasma gallisepticum DNA obtained in (1) described above was digested with XbaI, the digestion product was subjected to 0.6% low melting agarose gel electrophoresis. After the electrophoresis, the gel was immersed in an alkaline denaturation solution (0.5 M NaOH, 1.5 M NaCl) for 10 minutes to denature DNA and further immersed in a neutralizing solution (3 M sodium acetate, pH 5.5) for 10 minutes to neutralize. Following the neutralization, the DNA was transferred onto a nylon membrane in 6-fold SSC solution (0.7 M NaCl, 0.07 M sodium citrate, pH 7.5). After air drying, the membrane was heated at 80°C for 2 hours. 4-Fold SET (0.6 M NaCl, 0.08 M Tris-HCl, 4 mM EDTA, pH 7.8)-10-fold Denhardt-0.1% SDS-0.1% Na₄P₂O₇-50 µg/ml of denatured salmon sperm DNA and pUM-1 (see Japanese Patent Application Laid-Open No. 2-111795) which had been labelled in a conventional manner were added to cause hybridization at 68°C for 14 hours. The nylon membrane was overlaid on an X ray film. Autoradiography revealed that hybridization occurred on the fragment of about 3.4 kbp.

### (3) Cloning of XbaI-digested fragment of about 3.4 kbp to pUC-19 and colony hybridization

After 4 µg of Mycoplasma gallisepticum DNA obtained in Example 1 (1) described above was digested with restriction enzyme XbaI, the digestion product was subject to 0.6% low melting agarose gel electrophoresis. After the electrophoresis, the fragment of about 3.4 kbp was recovered. The fragment was ligated with XbaI-digested pUC-19 using ligase and competent E. coli TG1 strain was transformed by the ligation product. The transformants were cultured at 37°C for 15 hours in LB agar medium containing 0.003% of 5-bromo-4-chloro-3-indolyl-β-D-galactopyranoside, 0.03 mM of isopropylthio-β-D-galactopyranoside and 40 µg/ml of ampicillin. White colonies grown on the agar medium were transferred onto a nylon membrane followed by hybridization in a manner similar to (2) above. Autoradiography revealed that cloning was effected and, the thus obtained plasmid was named pUTTM1.

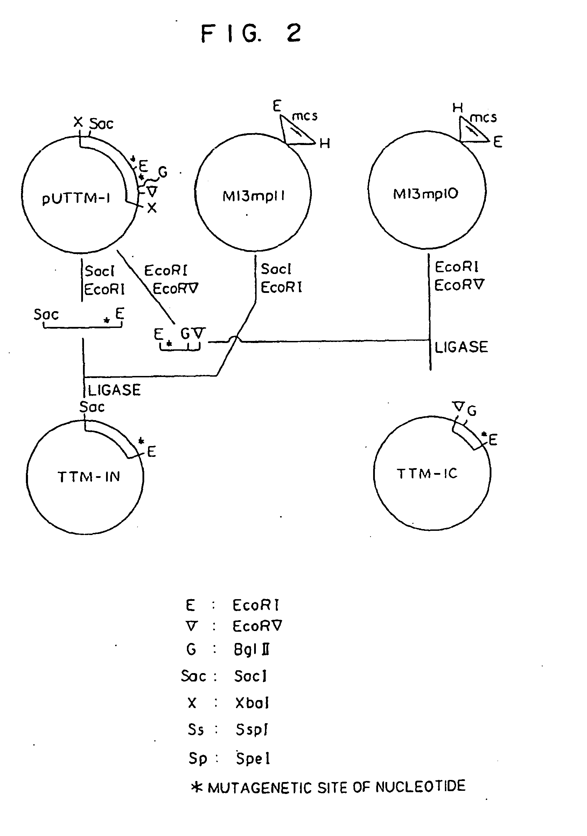

### (4) Production of TTM-1' modified (TGA → TGG) not to read TTM-1-encoding protein TTMG-1 by TGA as translation termination codon (see Fig. 2)

After pUTTM-1 of (3) described above was digested with restriction enzymes SacI and EcoRI and the digestion product was then subjected to 0.8% low melting agarose gel electrophoresis. The 1.1 kbp fragment containing the 5'-end of TTM-1 was recovered by treating with phenol-chloroform and precipitating with ethanol. The fragment was ligated with the fragment obtained by digestion of M13mp11 phage with SacI and EcoRI. The ligation reaction solution was mixed at m.o.i. of 0.1 with a solution obtained by culturing E. coli TG1 at 37°C for 24 hours, adding IPTG thereto in a final concentration of 100 mM and further supplementing IPTG in a X-gal concentration of 2%. The resulting mixture was inoculated on soft agar for solidification. Incubation was then performed at 37°C for 24 hours. Among the phage plaques formed, recombinant phage TTM-1N containing 1.1 kbp DNA of TTM-1 was collected from the phage in which the color did not change to blue.

Likewise, pUTTM-1 was digested with EcoRI and EcoRV. After 0.8% low melting agarose gel electrophoresis, the 0.4 kbp fragment containing the 3'-end of TTM-1 was recovered from the gel. A phenol-chloroform treatment followed by ethanol precipitation grave M13mp10 phage. M13mp10 phage was ligated with the fragment obtained by digestion with EcoRI and EcoRV using ligase. The reaction solution was treated as in the cloning of the 1.1 kbp DNA. Recombinant phage TTM-1C containing 0.4 kbp DNA of TTM-1 was thus obtained.

### (5) Preparation of single stranded DNA from each recombinant phage

The two recombinant phage obtained in (4) described above were added at m.o.i. of 0.1, respectively, to E. coli TG1 proliferated at 37°C in 100 ml of 2 x YT medium. After shake culture at 37°C for 5 hours, centrifugation was performed at 5000G for 30 minutes to obtain the cell-free supernatant. A 0.2-fold volume of polyethylene glycol/sodium chloride mixture (20% polyethylene glycol #6000, 2.5 M NaCl) was added to the supernatant. After settlement at 4°C for an hour, the mixture was centrifuged at 5000G for 20 minutes to recover the precipitates. The precipitates were dissolved in 500 µl of TE buffer (10 mM Tris-HCl, mM EDTA, pH 8.0). After extraction with phenol-chloroform, single stranded DNA of each recombinant phage was recovered by ethanol precipitation.

### (6) Construction of site-specific mutated plasmids using artificially synthesized oligonucleotide as a primer

The thus obtained DNA has TGA at the middle of the sequence. This TGA sequence is recognized as a termination codon in a normal cell so that the TGA sequence does not translate the sequence added thereafter. Therefore, in order to translate the TGA portion as methionine, the basic adenine which corresponds to the third nucleotide in codon NNN must be modified to guanine. Thus, the following two oligonucleotides were synthesized.
Sequence No. 17:
   3'-TACGTTCTTCCTGGCAAACCTTACCACTACTT-5'
Sequence No. 18:
   3'-CTACAAAGAACCTAAATATCA-5'

The oligonucleotide shown by Sequence No. 17 (SEQ ID NO: 17) is annealed to single stranded DNA of TTM-1N and the oligonucleotide shown by Sequence No. 18 to single stranded DNA of TTM-1C to cause the desired mutation by the method of Frits Eckstein et al. (Nucleic Acid Research, 8749-8764, 1985). The thus obtained recombinant phages were named TTM-1N' and TTM-1C', respectively. The TTM-1N' and TTM-1C' phage DNAs thus obtained were digested with restriction enzymes SacI-EcoRI and EcoRI-BglII, respectively. By 0.8% low melting agarose gel electrophoresis, the fragments of 1.1 kbp and 0.4 kbp were extracted from the agarose gel and recovered by ethanol precipitation. On the other hand, plasmid pUTTM-1 was also digested with SacI-BglII. The 4.8 kbp fragment bearing a vector was extracted by 0.8% low melting agarose gel electrophoresis and recovered by ethanol precipitation. The thus obtained three fragments were ligated by ligase and competent E. coli TG1 strain was transformed to obtain plasmid pUTTM-1' bear-ing TTM-1' with mutagenesis at the desired site thereof. The nucleotide sequence of TTM-1' is as shown by SEQ ID NO: 14 according to the Dideoxy method by Sanger et al. (Proc. Natl. Acad. Sci. USA, 74, 5463 (1977)). The nucleotide sequence is substantially the same as the 40 kilodalton TTM-1 polypeptide of M. gallisepticum.

### Reference Example 2

### Construction of vector pNZ1729R for insertion

The ECORI fragment (about 7.3 kbp) of NP strain was inserted into pUC18 at the EcoRI digestion site (terminus at the multi-cloning site) to obtain plasmid pNZ133 (about 10.0 kbp). From the plasmid the HpaI-SpeI fragment (about 3.0 kbp fragment derived from NP strain) was excised out and rendered blunt end by Klenow fragment. Furthermore, the EcoRI-HindIII fragment (multi-cloning site of 52 bp) was removed from pUC18 and rendered blunt end by Klenow fragment. The two fragments were ligated with each other to form a plasmid. After removing the EcoRV site in the BpaI-SpeI fragment, the EcoRI-HindIII fragment (multi-cloning site of 52 bp) of pUC18 is inserted therein using HindIII linker (5'-CAAGCTTG-3') and EcoRI linker (5'-GGAATTCC-3') to construct plasmid pNZ133SR.

Sequence No. 2 (SEQ ID NO: 2) and Sequence No. 3 (SEQ ID NO: 3) (bearing FPV promoter of 17 bases and linked to a translation initiation codon for lacZ) were annealed to double strands. Sequence No. 4 (SEQ ID NO: 4) annealed to the lacZ gene (derived from pMC1871 an pMA001, Sirakawa et al., Gene, 28, 127-132, 1984) and Sequence No. 5 (SEQ ID NO: 5), Sequence No. 6 (SEQ ID NO: 6) and Sequence No. 7 (SEQ ID NO: 7), Sequence No. 8 (SEQ ID NO: 8) and Sequence No. 9 (SEQ ID NO: 9), Sequence No. 10 (SEQ ID NO: 10) and Sequence No. 11 (SEQ ID NO: 11), were ligated with each other (which contains a modified synthetic promoter of poxvirus shown by nucleotide sequence: T T T T T TTTTTTTTTTTTTTTGGCATATAAA TAATAAATACAATAATTAATTACGC G T A A A A A T T G A A A A A C T A T T C T A A T TTATTGCACTC from the next T of AGC at the 5' end of Sequence No. 3 to C before G of Sequence No. 5 at the 3' end, and further linked to the multi-cloning site and poxvirus initial transcription termination signal on the both directions (SEQ ID NO: 12) (Yuen et al., Proc. Natl. Acad. Sci., USA, 88, 6417-6421, 1989) thereby to obtain the EcoRI-HindIII fragment (about 3.5 kbp). The EcoRI-HindIII fragment was inserted into pNZ133SR to construct plasmid pNZ1729R.

### Example 1

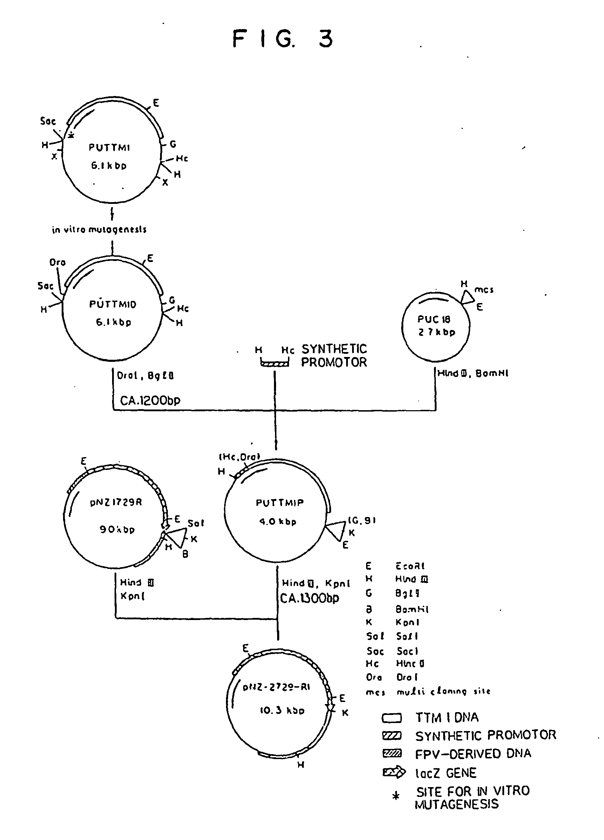

### Construction of plasmid pNZ7929-R1 for recombination (see Fig. 3)

### (1) Construction of plasmid pUTTMlP having ligated a synthetic promoter with TTM-1' gene

In order to form the restriction enzyme DraI digestion site upstream ATG corresponding to initiation codon of TTM-1' protein in plasmid pUTTM1' (see WO 93/24646) containing the full length TTM-1' DNA obtained in Reference Example 1, the following oligonucleotide was firstly prepared.
Sequence No. 19
   3'-TATAGAATTAAATTTTACTTATTC-5'

Next, after pUTTM-1' was digested with restriction enzymes SacI and EcoRI, the fragment of about 2300 bp was recovered and then ligated with the fragment obtained by digestion of M13mp10 with SacI and EcoRI to obtain recombinant phage TTM-1'. The oligonucleotide described above was annealed to single stranded TTM-1' to cause the desired variation by the method of Frits Eckstein et al. This recombinant phage DNA variant was digested with restriction enzymes SacI and EcoRI. The fragment of about 2300 bp was recovered and cloned to the vector-bearing fragment obtained by digestion of pUTTM-1' again with SacI and EcoRI to obtain pUTTMID.

A synthetic promoter was prepared by synthesizing DNAs of Sequence-20 and Sequence-21 followed by annealing, whereby the digestion sites with restriction enzymes HindIII and HincII at the end.
Sequence-20 5'-AGCTTTTTTTTTTTTTTTCCCATATAAATAATAAATACAATAATATACCCTAAAATT
Sequence-21

3'- AAAAAAAAAAAAAAAAAAAAAACCGTATATTTATTATTTATGTTATTAATTAATGCCGCATTTTTAA
HindIII

GAAAAACTATTCTAATTTATTGCACTCGTC -3'
CTTTTTGATAAGATTAAATAACGTGAGCAG -5'
HincII

Finally, the 1200 bp fragment obtained by digestion of pUTTMID with restriction enzymes DraI and BglII was ligated with the synthetic promoter described above and the fragment obtained by digestion of pUC18 with HindIII and BamHI to give plasmid pUTTMIP of about 4.0 kbp.

### (2) Construction of pNZ7929R1

After plasmid pUTTMlp obtained in (1) was digested with restriction enzymes HindIII and KpnI, the fragment of about 1300 bp was recovered. Next, vector pNZ1729R (EP-A-0520753) for FPV recombination obtained in Reference Example 2 was digested with restriction enzymes HindIII and KpnI. The two fragments were ligated with each other to obtain the desired vector pNZ7929-R1 (about 10.3 kbp) for recombination.

### (3) Construction of recombinant FPV fNZ7929-R11 and purification thereof

NP strain, which is a fowlpox live vaccine strain, was infected to monolayered CEF at m.o.i. = 0.1. Three hours after, these cells were peeled apart from the monolayer by a treatment with trypsin to form a cell suspension. After 2 x 10⁷ cells in the suspension were mixed with 10 µg of plasmid pNZ7929-R1 for recombination, the mixture was suspended in Saline G (0.14M NaCl, 0.5 mM KCl, 1.1 mM Na₂HPO₄, 1.5 mM K9₂PO₄, 0.5 mM MgCl₂ 6H₂O, 0.011% glucose). The suspension was subjected to electrophorasis under conditions of 3.0 kV cm⁻¹, 0.4 msec and 25°C, using Gene Pulser (Bio-Rad) at room temperature. The plasmid-infected cells were then cultured at 37°C for 72 hours. The cells were lysed by freeze and thaw 3 times to recover viruses containing the recombinant virus.

The recombinant virus recovered was selected as follows. The recovered viral solution was infected to monolayered CEF and 10 ml of agar solution containing growth medium was overlaid thereon. After agar was solidified at room temperature, incubation was performed at 37°C until plaques of FPV appeared. Then agar medium containing Bluo gal in a concentration of 200 µg/ml was overlaid on the agar followed by incubation at 37°C for further 48 hours. Among all of the plaques, about 1% of the plaques were colored blue. These blue plaques were isolated and recovered. By the same procedures, isolation and recovery were repeated to purify the virus until all the plaques were stained to blue with Bluo gal. In general, the repeated procedures were terminated by 3 to 4 times. The purified virus was named fNZ7929-R1. In fNZ7929-R1, each position of the DNA inserted was confirmed by dot blotting hybridization and Southern blotting hybridization.

### Reference Example 2 Obtaining of 70 K protein DNA

### (1)_{.} Preparation of Mycoplasma gallisepticum genomic DNA

Using Mycoplasma gallisepticum S6 strain, 200 µg of Mycoplasma gallisepticum DNA was obtained in a manner similar to Reference Example 1 (1) described above.

### (2) Preparation of genomic DNA library

After 4 units of restriction enzyme AluI was added to 40 µg of Mycoplasma gallisepticum genomic DNA obtained in (1), incubation was conducted at 37°C for 10 minutes for partial digestion. The partially digested genomic DNA was subjected to 0.8% low melting agarose gel electrophoresis. The DNA fragment having a strand length of approximately 1.0 kbp to 4.0 kbp was recovered from the gel. The DNA fragment was treated with phenol and then precipitated with ethanol to give 4 µg of the DNA fragment partially digested with AluI.

S-Adenosyl-L-methionine was added to 1.2 µg of the AluI-partially digested-DNA fragment in a final concentration of 80 µM and 20 units of EcoRI methylase was further added thereto to methylate the deoxy-adenosine site in the EcoRI recognition sequence, thereby to render the sequence non-sensitive to EcoRI. EcoRI linker was ligated with this DNA fragment using ligase. The ligation product was then mixed with the EcoRI digestion fragment of λgt11 DNA to ligate with each other by ligase. The reaction solution was used to effect in vitro packaging in a conventional manner (DNA Cloning, Vol. 1, A Practical Approach, edited by D.M. Glover). The resulting product was transfected to Escherichia coli Y1088 strain (Amersham) followed by incubation at 37°C for 12 hours in LB agar medium containing 0.003% of 5-bromo-4-chloro-3-indolyl-β-D-galactopyranoside and 0.03 mM isopropylthio-β-D-galactopyranoside. Among the plaques formed, a library size was estimated by the count of white plaques to prepare DNA library of 106 pfu (plaque forming unit).

### (3) Immuno-screening of genomic DNA library

The phage obtained from the DNA library prepared in (2) was added to a suspension of Escherichia coli Y1090 strain (Amersham) in an aqueous solution of 10 mM MgSO₄ to form 500 to 1000 plaques on one plate of 8 cmφ to effect adsorption for 15 minutes. Furthermore 2.5 ml of LB soft agar medium warmed to 45°C was added and overlaid on the LB agar medium followed by incubation at 42°C for 3 to 4 hours. A nylon membrane filter was immersed in 10 mM of IPTG aqueous solution, air-dried and then overlaid on the plate described above followed by incubation at 37°C for further 2 to 3 hours. After the incubation, the nylon membrane filter was peeled apart from the plate and washed with TBS (50 mM Tris-HCl, pH 8.0, 150 mM NaCl). The filter was immersed in 2% skimmed milk-containing TBS for 30 minutes and then treated for an hour with anti-Mycoplasma chicken serum diluted with TBS to 500-fold. Thereafter, the filter was immersed in TBS for 15 minutes to wash the filter. The filter was further washed by immersing in TBS containing 0.05% of a surfactant (Tween 20) for 10 to 15 minutes. This step was repeated 4 to 5 times. Then the filter was treated with biotinylated antibody against chicken IgG for 60 minutes. After treating with a secondary antibody, the filter was washed with PBS containing 0.05% of Tween 20 5 to 6 times and then treated for 60 minutes by immersing in horse radish peroxidase-avidin D solution. After the treatment, the filter washed with PBS containing 0.05% of Tween 20 5 to 6 times and then washed with 10 mM Tris-HCl, pH 8.0. Then, the filter was immersed in a buffer containing 4-chloronaphthol and hydrogen peroxide. By a series of these operations, only the plaques that expressed an antigenic protein originating in Mycoplasma gallisepticum were colored purple.

By the aforesaid immuno-screening of about 5 x 10⁴ plaques, 50 positive plaques were obtained.

### (4) Production of immuno-positive recombinant λgt11 phage DNA

Escherichia coli Y1090 strain was incubated at 37°C for 12 hours in LB medium supplemented with 50 µg/ml ampicillin. The culture broth was added to a 10-fold amount of LB medium containing mM MgSO₄. Then, recombinant λgt11 phage which was obtained in (3) and became positive by immuno-screening was added to the medium at m.o.i. = 0.05, followed by incubation at 37°C for 5 to 10 hours. After lysis of Escherichia coli, centrifugation was carried out at 8,000 rpm for 10 minutes to obtain the supernatant. To the supernatant were added an equal volume of TM buffer (50 mM Tris-HCl, pH 7.4, 10 mM MgSO₄) and DNase I in a concentration of 0.016 mg/ml, followed by incubation for 15 minutes. After NaCl and polyethylene glycol (PEG 6000) were added to the culture broth in concentrations of 0.5 M and 0.1 g/ml, respectively, the mixture was shaken at 0°C for 15 minutes. After centrifugation at 10,000 rpm for 10 minutes, the supernatant was removed. The resulting pellets were dissolved in a 1/100 volume of TM buffer and an equal volume of chloroform was added thereto followed by vigorous stirring. By centrifugation at 15,000 rpm for 10 minutes, recombinant λgt11 phage was collected in the aqueous phase to obtain the phage solution.

EDTA, SDS and pronase E were added to the phage solution in final concentrations of 0.025 M, 1% and 1 mg/ml, respectively. After incubation at 37°C for 4 hours, the solution was subjected to phenol extraction and ethanol precipitation to give λgt11 phage DNA containing the cloned antigenic DNA (M-81).

### (5) Construction of recombinant plasmid (pM-81)

The recombinant λgt11 phage DNA obtained in (4) was digested with restriction enzyme EcoRI, the digestion product was subjected to 0.8% low melting agarose gel electrophoresis. The genomic DNA fragment of Mycoplasma gallisepticum inserted into the genomic DNA of λgt11 phage at the cloning site showed a strand length of about 2.8 kbp. This DNA fragment was extracted from the agarose gel and then with phenol-chloroform (1:1) and recovered by ethanol precipitation. On the other hand, after plasmid pUC18 was digested with EcoRI, the digested pUC18 was extracted with phenol-chloroform and recovered by ethanol precipitation, in a similar manner. Then, the phosphate at the 5' end was removed by an alkaline phosphatase treatment. After pUC18 DNA was again extracted with phenol-chloroform, DNA was recovered by ethanol precipitation.

The digested pUC18 was ligated with the EcoRI digestion product (about 0.8 kbp) derived from Mycoplasma gallisepticum using ligase. Competent Escherichia coli TG1 strain was transformed with the ligation product. The transformants were cultured at 37°C for 15 hours in LB agar medium supplemented with 0.003% of 5-bromo-4-chloro-3-indolyl-β-D-galactopyranoside, 0.03 mM of isopropylthio-β-D-galactopyranoside and 40 µg/ml of ampicillin. Among the transformed E. coli grown on the agar medium, white colonies were cultured at 37°C for 15 hours in 40 µg/ml ampicillin-supplemented LB liquid medium and plasmid was extracted by the method of Birnboin & Doly (Nuc. Acid Res., 7, 1513 - (1979)]. After digestion with EcoRI, the recombinant plasmid containing the same length of DNA fragment as that of the original EcoRI fragment derived from Mycoplasma gallisepticum was detected by 0.8% low melting agarose electrophoresis; this plasmid was named pM-81.

### (6) Genomic Southern hybridization of Mycoplasma gallisepticum using M-81 DNA as a probe

After 1 µg of pM81 obtained in (5) described above was digested with EcoRI and HindIII, the digestion product was subjected to 0.6% low melting agarose gel electrophoresis. After the electrophoresis, the gel was immersed in an alkaline denaturation solution (0.5 M NaOH, 1.5 M NaCl) for 10 minutes to denature DNA. The gel was then immersed in a neutralization solution (3 M sodium acetate, pH 5.5) for 10 minutes for neutralization and then transferred onto a nylon membrane in 6-fold SSC solution (0.7 M NaCl, 0.07 M sodium citrate, pH 7.5). After air-drying, the nylon membrane was baked at 80°C for 2 hours and 4-fold SET (0.6 M NaCl. 0.08 M Tris-HCl, 4 mM EDTA, pH 7.8)-10-fold Denhardt-0.1% SDS-0.1% Na₄P₂O₇-50 µg/ml of denatured salmon sperm DNA and pM-81 (M-81 gene is contained in this plasmid) labelled in a conventional manner was added thereto to perform hybridization at 68°C for 14 hours. The nylon membrane was overlaid on an X ray film. It was confirmed by autoradiography that M-81 was hybridized to the about 5.0 kbp fragment of Mycoplasma gallisepticum.

### (7) Cloning of EcoRI and HindIII-digested fragment of about 5.0 kbp to pUC19 and colony hybridization

After 4 µg of the Mycoplasma gallisepticum DNA obtained in (6) described above was digested with EcoRI and HindIII, the digestion product was subjected to 0.6% low melting agarose gel electrophoresis to recover the fragment of about 5.5 kbp. The fragment was ligated with pUC-19 cleaved by digestion with EcoRI and HindIII using ligase. Competent Escherichia coli TG1 strain was transformed with the ligation product. The transformants were cultured at 37°C for 15 hours in LB agar medium supplemented with 0.003% of 5-bromo-4-chloro-3-indolyl-β-D-galactopyranoside, 0.03 mM of isopropylthio-β-D-galactopyranoside and 40 µg/ml of ampicillin. White colonies grown on the medium were transferred onto a nylon membrane and hybridization was carried out in a manner similar to (2) described above. It was confirmed by autoradiography that cloning was effected and this plasmid was named pUM-81.

### (8) Sequence analysis of pUM-81 insert DNA

The sequence of about 5.0 kbp fragment inserted into pUM-81 prepared in (7) above was analyzed by the dideoxy method by Sanger et al.

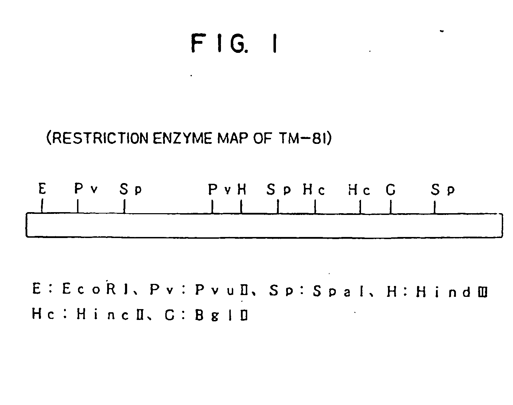

The restriction enzyme map of the open reading frame (hereinafter abbreviated as ORF) present in this fragment is shown in Fig. 1. The nucleotide sequence of this ORF and the amino acid sequence deduced therefrom are shown by SEQ ID NO: 1. The polypeptide deduced from this ORF was named TM-81 polypeptide.

### Reference Example 3

### Construction of recombinant FPV bearing hybrid DNA in which TTM-1' protein DNA was ligated downstream the signal membrane anchor DNA

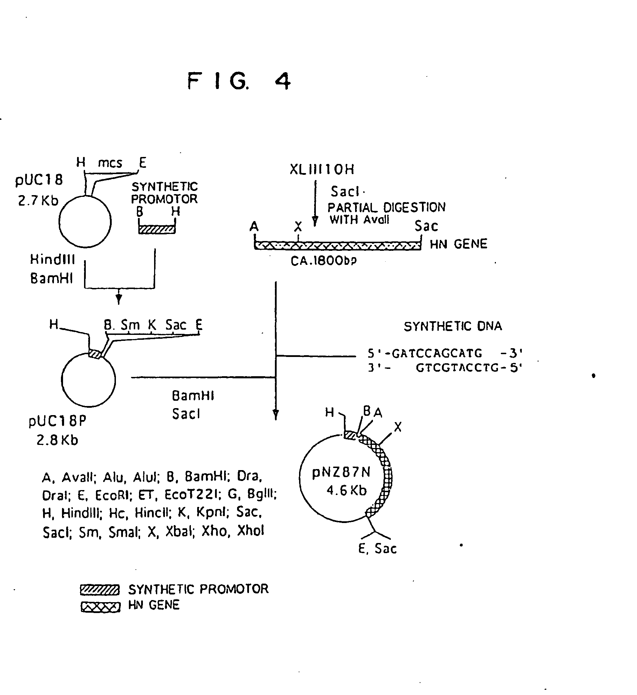

### (1) Cloning of the synthetic promoter to pUC18 (see Fig. 4)

The following synthetic promoters bearing the HindIII and BamHI restriction enzyme sites at both ends were synthesized.

HindIII

Sequence No. 22 5'- AGCTTTTTTTTTTTTTTTTTTTTGGCATATAAATAATAAATACAATAATTAATTACGC

Sequence No. 23 3'- AAAAAAAAAAAAAAAAAAAAACCGTATATTTATTATTTATGTTATTAATTAATGCG

GTAAAAATTGAAAAACTATTCTAATTTATTGCACTCG -3'

CATTTTTAACTTTTTGATAAGATTAAATAACGTGAGCCTAG -5'

BamHI

This synthetic DNA was ligated with the digestion fragment of PUC18 with HindIII and BamHI to obtain plasmid of about 2.8 kbp named PUC18P.

### (2) Ligation of a gene encoding HN protein of NDV with the synthetic promoter (see Fig. 4)

After plasmid XLIII-10H bearing HN gene of NDV was fully digested with SacI, the digestion product was then partially digested with AvaII. The fragment of about 1800 bp was recovered by 0.8% low melting agarose gel electrophoresis. In order to form the BamHI cleavage site at the AvaII site of this fragment, the following DNA was synthesized.

BamHI AvaII
Sequence-24 5' - GATCCAGCATG - 3'
Sequence-25 3' - GTCGTACCTG - 5'

Three of the synthetic DNA, the HN-bearing DNA fragment of about 1800 bp and the fragment containing the synthetic promoter recovered by 2.0% low melting agarose gel electrophoresis after full digestion of pUC18P with BamHI and SacI were ligated by ligase and these three fragments-ligated plasmid was extracted. The resulting plasmid of about 4.6 kbp was named pNZ87N.

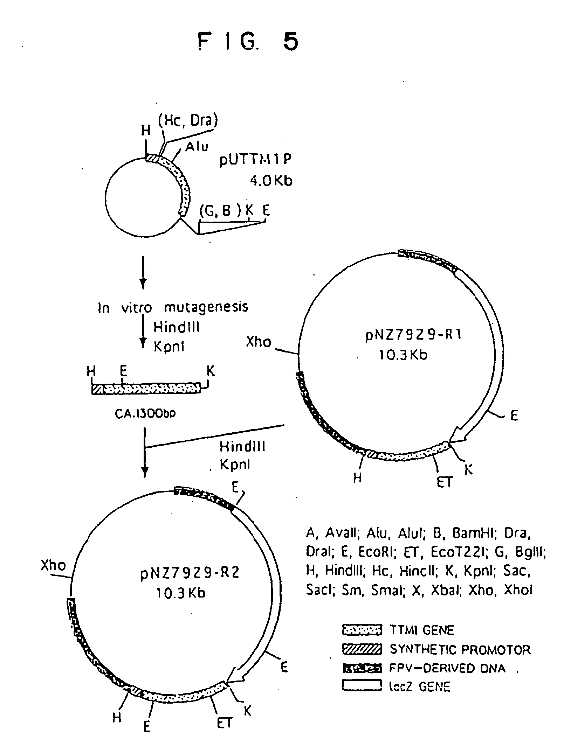

### (3) Change of the AluI cleavage site of pNZ7929-R1 into the EcoRI cleavage site (see Figs. 3 and 5)

In order to change the restriction enzyme AluI cleavage site in the 279 nucleotide portion of SEQ ID NO: 14 into the EcoRI cleavage site, the following oligonucleotide was synthesized.
Sequence-26 5'-GGGATTTCGAATTCTATGTCT-3 '

After pUTTM1P was digested with HindIII and KpnI, the fragment of about 1300 bp and ligated with the fragment obtained by digestion of M13mp10 with HindIII and KpnI to obtain the single stranded recombinant phage. The oligonucleotide described above was annealed to the single stranded recombinant phage to cause the desired mutation by the method of Frits Eckstein et al. After the recombinant phage DNA mutant was digested with restriction enzymes HindIII and KpnI, the fragment of about 1300 bp was recovered and ligated with the fragment obtained by digestion of pNZ1729R with restriction enzymes HindIII and KpnI using ligase to obtain plasmid pNZ7929-R2 (about 10.3 kbp) with the AluI cleavage site of pNZ7929-R1 being changed into the EcoRI cleavage site.

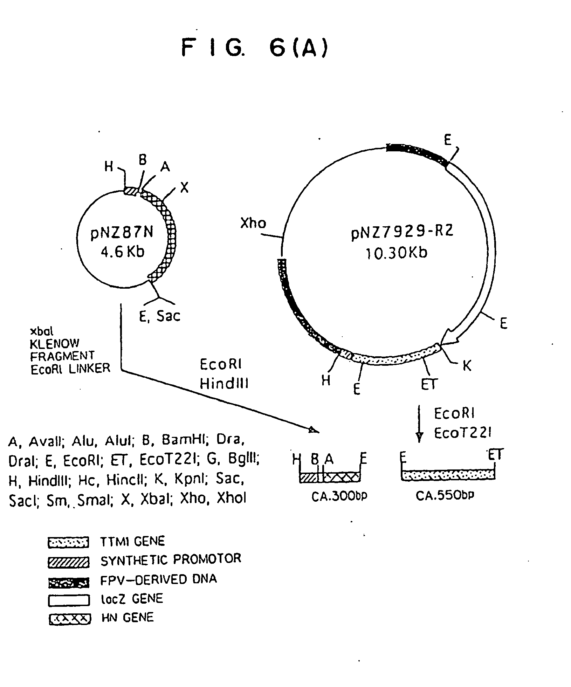

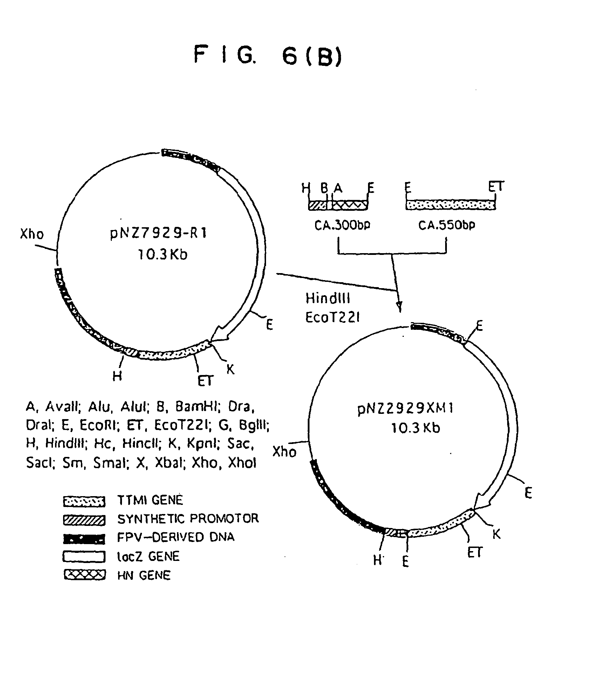

### (4) Construction of plasmid pNZ2929XM1 for recombinant FPV (see Fig. 6(A) and 6(B))

Firstly pNZ87N was fully digested with restriction enzyme XbaI and the cleavage site was rendered blunt by Klenow fragment. Then EcoRI linker (5'-GGAATTCC-3') was added to and ligated with the digestion product using ligase. The plasmid was digested with EcoRI and HindIII. The fragment of about 300 bp was recovered by 1.2% low melting agarose gel electrophoresis. Next, pNZ7929R2 was digested with restriction enzyme EcoT22I and then partially digested with EcoRI. The fragment of about 550 bp which is a part of TTM-1 DNA was recovered by 0.8% low melting agarose gel electrophoresis. Furthermore, pNZ7929RI was digested with restriction enzymes EcoT22I and HindIII and the fragment of about 9.4 kbp was recovered by 8% low melting agarose gel electrophoresis. These fragments were ligated by ligase and the three fragments-ligated plasmid was extracted. The plasmid of about 10.3 kbp was named pNZ2929XM1.

### (5) Construction and purification of recombinant FPV fNZ2929XM1

Construction and purification were carried out in a manner similar to Example 1 (3). The purified virus was named fNZ2929-XM1. By dot blotting hybridization and Southern blot hybridization, the location of each DNA inserted was confirmed in fNZ2929-XMI.

### Reference Example 4

### Expression of TTM-1 polypeptide in cells infected with fNZ7929-R1 and fNZ2929XM1

In order to confirm that fNZ7929-R1 and fNZ2929XM1 express TTM-1 polypeptide in infected cells, immunofluorescent antibody technique using antisera against Mycoplasma gallisepticum S6 was employed. fN7929-R1 and fNZ2929XM1 were infected to CEF and incubation was carried out at 37°C until plaques appeared. After fixing with cold acetone, chicken antisera (anti-S6) immunized with Mycoplasma gallisepticum S6 strain or Mycoplasma gallisepticum S6-infected chicken sera (S6 infected) and TTM-1 polypeptide-immunized chicken antisera (anti-TTMG-1) diluted as a primary antibody to 100 to 1000-fold were reacted. These culture cells were further reacted with anti-chicken immunoglobulin bound to a fluorescent substance (FITC) and the non-specific reaction portion was washed out. Then, microscopic observation was made under the excited fluorescent wavelength light. With respect to the infected cells where no acetone fixing was performed (namely, unfixed cells), the reactivity was likewise examined. Using FPV-NP strain and fNZ2337 (Japanese Patent Application Laid-Open No. 1-157381) as viruses for control, Newcastle disease virus-immunized chicken sera (anti-NDV) and SPF chicken sera (SPF) were used in 1000-fold as primary antibody for control. The reactivity is shown in Table 1.

**Table 1. Reactivity of recombinant virus-infected CEF to various antisera**

| Reactivity to primary antibody Infected | | | | | |
|---|---|---|---|---|---|
| Infected virus | | | | | |
| (acetone fixation) | anti-S6 | S6 | anti-TTM-1 | anti-NDVSPF | |
| fNZ2929XM1 | | | | | |
| (acetone-fixed) | ++ | ++ | ++ | - | - |
| (non-fixed) | + | + | + | - | - |
| fNZ7929-R1 | | | | | |
| (acetone-fixed) | + | + | | - | - |
| (non-fixed) | ± | ± | ± | - | - |
| fNZ2337 | | | | | |
| (acetone-fixed) | - | - | - | + | - |
| (non-fixed) | - | - | - | + | - |
| NP | | | | | |
| (acetone-fixed) | - | - | - | - | - |
| (non-fixed) | - | - | - | - | - |
| None | | | | | |
| (acetone-fixed) | - | - | - | - | - |
| (non-fixed) | - | - | - | - | - |

| | | | | | |
|---|---|---|---|---|---|
| ++ : strongly reacted + : reacted ± : weakly reacted - : not reacted | | | | | |

The results reveal that the cells infected with the recombinant viruses fNZ7929-R1 and fNZ2929XM1 of the present invention are reactive with anti-S6, S6 infection and anti-TTM-1; and that fNZ7929-R1 are reactive with anti-56, S6 infection and anti-TTMG-1 also in non-fixed completed cells. This indicates that fNZ2929XM1 not only expresses TTMG-1 polypeptide in the infected cells but also exhibits TTM-1 polypeptide on the surface of the infected cells.

### Reference Example 5

### Antibody inducing ability of recombinant FPV inoculated to chickens

After fNZ7929-R1 and fNZ2929XM1 were cultured in CEF at 37°C for 48 hours, the procedure of freezing and thawing was repeated twice to recover the cell suspension. The cell suspension was adjusted to have a virus titer of 106 pfu/ml and then inoculated to SPF chick (Line M, Nippon Seibutsu Kagaku Kenkyusho) of 7 days old at the right wing web in a dose of 10 µl. After the inoculation, generation of the pock was observed. Two weeks after the inoculation, sera were collected. The antibody titer of the sera collected was determined by ELISA. The purified TTM-1 polypeptide was dissolved in bicarbonate buffer in a concentration of 1 µg/well. After adsorbing to a 96 well microtiter plate, blocking was performed with skimmed milk to prevent the following non-specific adsorption. Next, a dilution of the sample serum was charged in each well and then horse radish peroxide-bound anti-chick immunoglobulin antibody (rabbit antibody) was added thereto as a secondary antibody. After thoroughly washing, 2,2'-azinodiethylbenzothiazoline sulfonate was added to the mixture as a substrate and a relative dilution magnification of the antibody was measured with an immuno-reader in terms of absorbance at a wavelength of 405 nm. As a primary antibody for control, anti-TTM-1 polypeptide chicken serum was used. The results are shown in Table 2.

**Table 2. Antibody titer of fNZ2929XM1-inoculated chick to TTM-1 polypeptide**

| Inoculated virus | Antibody titer to anti-TTM-1 polypeptide (dilution magnification)* |
|---|---|
| fNZ2929XM1 | 256 |
| fNZ7929-R1 | 32 |
| NP | 1 |
| ** | 1 |
| anti-TTM-1 polypeptide | 256 |

| | |
|---|---|
| * dilution magnification when SPF chicken serum dilution is as 1 ** not inoculated | |

The results reveal that both fNZ2929XM1 and fNZ2929-R1 which are recombinant viruses, can induce anti-TTN-1 polypeptide antibody and can be used as a vaccine for effectively preventing fowlpox and Mycoplasma gallisepticum infections.

### Example 6

### Collection of recombinant Avipox virus fNZ7929-67 bearing TM-67

### (1) Genomic Southern hybridization of Mycoplasma gallisepticum using TM-67 gene as a probe

After 1 µg of the Mycoplasma gallisepticum DNA obtained in Reference Example (1) was digested with XbaI, the digestion product was subjected to 0.6% low melting agarose gel electrophoresis. After the electrophoresis, the gel was immersed in an alkaline denaturation solution (0.5 M NaOH, 1.5 M NaCl) for 10 minutes to denature DNA. The gel was then immersed in a neutralization solution (3 M sodium acetate, pH 5.5) for 10 minutes for neutralization and then transferred onto a nylon membrane in 6-fold SSC solution (0.7 M NaCl, 0.07 M sodium citrate, pH 7.5). After air-drying, the nylon membrane was baked at 80°C for 2 hours and 4-fold SET (0.6 M NaCl, 0.08 M Tris-HCl, 4 mM EDTA, pH 7.8)-10-fold Denhardt-0.1% SDS-0.1% Na₄P₂O₇-50 µg/ml of denatured salmon sperm DNA and pUM-1 (cf. Japanese Patent Application Laid-Open No. 2-111795) labelled in a conventional manner was added thereto to perform hybridization at 68°C for 14 hours. The nylon membrane was overlaid on an X ray film. It was confirmed by autoradiography that hybridization occurred to the about 3.4 kbp fragment different from the fragment confirmed in Reference Example 1 (2).

### (2) Cloning of the XbaI-digested fragment of about 3.4 kbp to pUC-19 and analysis of the sequence

After 4 µg of the Mycoplasma gallisepticum DNA obtained in Reference Example 1 (1) was digested with restriction enzyme XbaI, the digestion product was subjected to 0.6% low melting agarose gel electrophoresis to recover the fragment of about 3.4 kbp confirmed in Example 6 (1) described above. The fragment was ligated with pUC-19 cleaved by digestion with XbaI using ligase. Competent Escherichia coli TG1 strain was transformed with the ligation product. The transformants were cultured at 37°C for 15 hours in LB agar medium supplemented with 0.003% of 5-bromo-4-chloro-3-indolyl-B-D-galactopyranoside, 0.03 mM of isopropylthio-B-D-galactopyranoside and 40 µg/ml of ampicillin. Among the transformed E. coli grown on the medium, white colonies were cultured at 37°C for 15 hours in LB liquid medium supplemented with 40 µg/ml ampicillin and plasmid was extracted by the method of Birnboim & Doly. After digestion with XbaI, the recombinant plasmid containing the same length as that of the XbaI fragment derived from MG was detected by 0.8% low melting agarose electrophoresis; this plasmid was named pUM67.

The about 3.4 kbp fragment inserted into pUM67 was analyzed by the dideoxy method by Sanger et al.

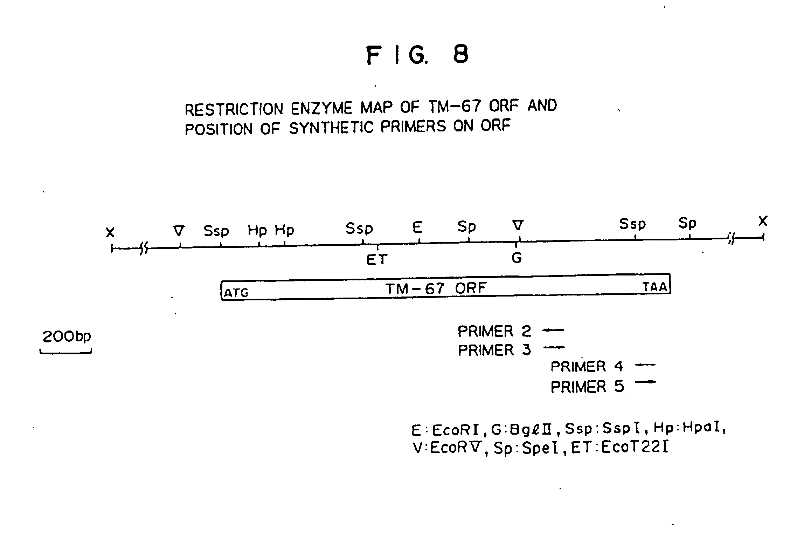

The restriction enzyme map of the open reading frame (ORF) present in this fragment is shown in Fig. 8 and the nucleotide sequence of this ORF and the amino acid sequence are shown by SEQ ID NO: 27. The polypeptide deduced from this ORF was named TM-67 polypeptide.

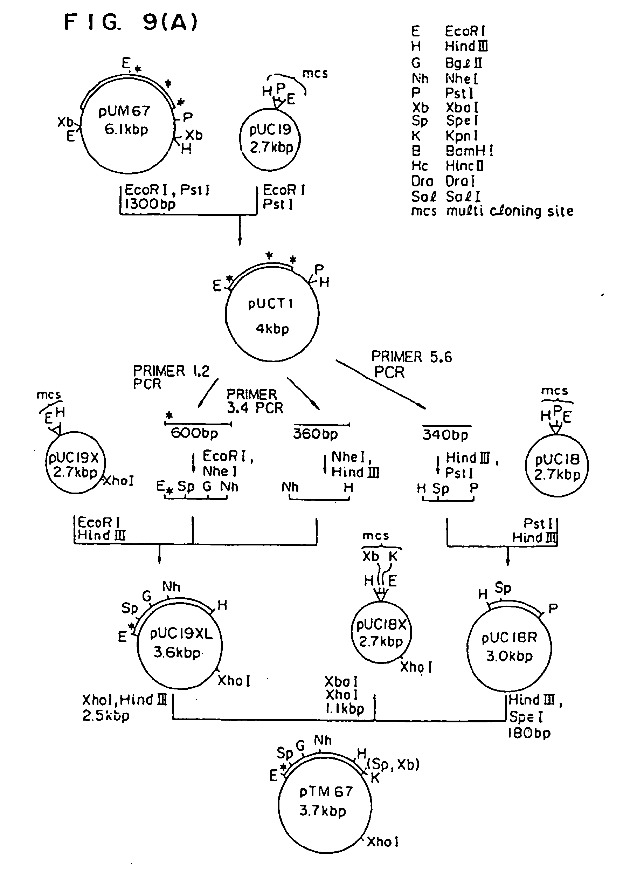

### (3) Construction of plasmid pTM67 bearing a modified gene (TGA + TGG) not to read TGA in ORF of TM-67 as translation termination codon (see Figs. 8 and 9(A))

TGA codons were concentrated at the downstream 10 portion in ORF of TM-67. Therefore, the EcoRI and PstI fragment of about 1300 bp containing all TGA codons were recovered from pUM67 and ligated with pUC19 digested with EcoRI and PstI to obtain PUCT1 (4.0 kbp). Next, in order to change TGA to TGG using PUCT1 as a template 15 according to polymerase chain reaction (PCR: Science, 230, 1350-1354 (1985)), primer DNAs for PCR shown by SEQ ID NOS: 28-33 were synthesized.

Primers 1 to 6 corresponding to SEQ ID NOS: 28-33 which were employed for PCR are as follows.
Primer-1 5'-GTTTTCCCAGTCACGAC-3' (M13 primer)

Primer-2  3'-AACCAACCAACCCGCGATCGCTAGTCT-5'

Nhe I

Primer-3  5'-TGATTGGGCGCTAGCGATCA-3'
Nhe I

Primer-4  3'-TCCCAACCTTGTTCGAAATACAA-5'
HindIII

Primer-5  5'-TGAAACAAGCTTTATGTTT-3'
HindIII

Primer-6 3'-CAGTATCGACAAAGGAC-5' (M13 RV primer)

Following the conventional procedures for PCR, the fragment of 600 bp was amplified using Primer-1 and Primer-2 and then recovered; the fragment of 360 bp using Primer-3 and Primer-4; and the fragment of 340 bp using Primer-5 and Primer-6. In addition, the fragment of 600 bp was digested with EcoRI and NheI; the fragment of 360 bp was digested with Nhe and HindIII; and the fragment of 340 bp was digested with HindIII and PstI. Thereafter each digestion product was subjected to 2.0% low melting agarose gel electrophoresis and recovered from the agarose. For cloning of each fragment, pUC19 and pUC18 were digested with DraI and then XhoI linker was inserted to obtain plasmids pUC19X and pUC18X. The fragment of 600 bp and the fragment of 360 bp treated with the respective restriction enzymes and recovered were ligated with the digestion product of pUC19X with EcoRI and HindIII by ligase. The resulting plasmid was extracted and this plasmid was named pUC19XL (about 3.6 kbp). The 340 bp fragment digested with HindIII and PstI was ligated with the fragment obtained by digesting pUC18 with HindIII and PstI, using ligase. The resulting plasmid was extracted and named pUC18R (about 3 kbp). The fragment of about 2.5 kbp obtained by digestion of pUCI9XL with HindIII and XhoI, the fragment of 180 bp obtained by digestion of pUC18R with HindIII and SpeI, and the fragment of 1.1 kbp obtained by digestion of pUC18X with XbaI and XhoI were subjected to agarose gel electrophoresis, respectively, and then recovered. These fragments were ligated using ligase. The resulting plasmid was extracted and named pTM67 (about 3.7 kbp).

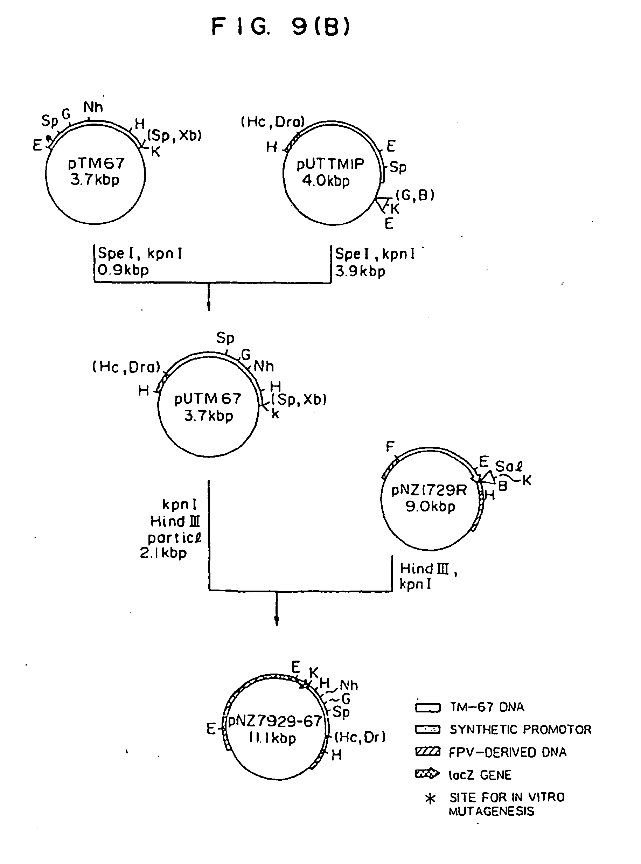

### (4) Construction of pNZ7929-67 (Fig. 9 (B))

After pUTTM1P obtained in Example 1 (1) was digested with SpeI and KpnI, the digestion product was subjected to agarose gel electrophoresis to recover the fragment of 3.9 kbp. In a similar manner, after pTM67 was digested with SpeI and KpnI, the digestion product was subjected to agarose gel electrophoresis to recover the fragment of 0.9 kbp. The thus recovered fragment was ligated with the 3.9 kbp fragment described above using ligase. The resulting plasmid pUTM67 (4.8 kbp) was recovered. After this pUTM67 was digested with KpnI, the digestion product was partially digested with HindIII. The product was then subjected to agarose gel electrophoresis to recover the fragment of 2.1 kbp. The thus recovered fragment was ligated with the 9.0 kbp fragment obtained by digestion of PNZ1729R (cf. Reference Example 2) with HindIII and KpnI, using ligase. The resulting plasmid pNZ7929-67 (11.1 kbp) was recovered.

### (5) Construction of recombinant Avipox virus fNZ7929-67 and purification

The procedures similar to Example 1 (3) were repeated using pNZ7929-67 obtained in (4) described above to obtain fNZ7929-67.

### Reference Example 7

### Collection of recombinant Avipox virus fNZ7929-66 bearing TM-66

### (1) Genomic Southern hybridization of Mycoplasma gallisepticum using TM-66 gene as a probe

After 1 µg of the Mycoplasma gallisepticum DNA obtained in Reference Example (1) was digested with XbaI, the digestion product was subjected to 0.6% low melting agarose gel electrophoresis. After the electrophoresis, the gel was immersed in an alkaline denaturation solution (0.5 M NaOH, 1.5 M NaCl) for 10 minutes to denature DNA. The gel was then immersed in a neutralization solution (3 M sodium acetate, pH 5.5) for 10 minutes for neutralization and then transferred onto a nylon membrane in 6-fold SSC solution (0.7 M NaCl, 0.07 M sodium citrate, pH 7.5). After air-drying, the nylon membrane was baked at 8.0°C for 2 hours and 4-fold SET (0.6 M NaCl, 0.08 M Tris-HCl, 4 mM EDTA, pH 7.8)-10-fold Denhardt-0.1% SDS-0.1% Na₄P₂O₇-50 µg/ml of denatured salmon sperm DNA and pUM-1 (cf. Japanese Patent Application Laid-Open No. 2-111795) labelled in a conventional manner was added thereto to perform hybridization at 68°C for 14 hours. The nylon membrane was overlaid on an X ray film. It was confirmed by autoradiography that hybridization occurred to the about 6.3 kbp fragment.

### (2) Cloning of the XbaI-digested fragment of about 6.3 kbp to pUC-19 and analysis of the sequence

After 4 µg of the My-coplasma gallisepticum DNA obtained in Reference Example 1 (1) was digested with restriction enzyme XbaI, the digestion product was subjected to 0.6% low melting agarose gel electrophoresis to recover the fragment.of about 6.3 kbp confirmed in Example 7 (1) described above. The fragment was ligated with pUC-19 cleaved by digestion with XbaI using ligase. Competent Escherichia coli TG1 strain was transformed with the ligation product. The transformants were cultured at 37°C for 15 hours in LB agar medium supplemented with 0.003% of 5-bromo-4-chloro-3-indolyl-β-D-galactopyranoside, 0.03 mM of isopropylthio-β-0-galactopyranoside and 40 µg/ml of ampicillin. White colonies grown on the medium were transferred to a nylon membrane and hybridization was carried out in a manner similar to (1) described above. Autoradiography reveals that cloning was effected and this plasmid was named pUM66 (about 9 kbp).

The about 6.3 kbp fragment inserted into pUM66 was analyzed by the dideoxy method by Sanger et al.

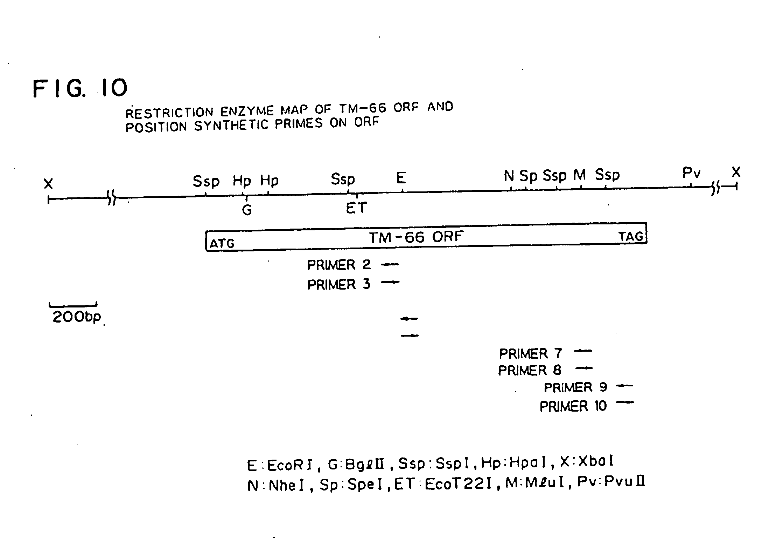

The restriction enzyme map of ORF present in this fragment is shown in Fig. 10 and the nucleotide sequence of this ORF and the amino acid sequence deduced therefrom are shown by SEQ ID NO: 16.

The polypeptide deduced from this ORF was named TM-66 polypeptide.

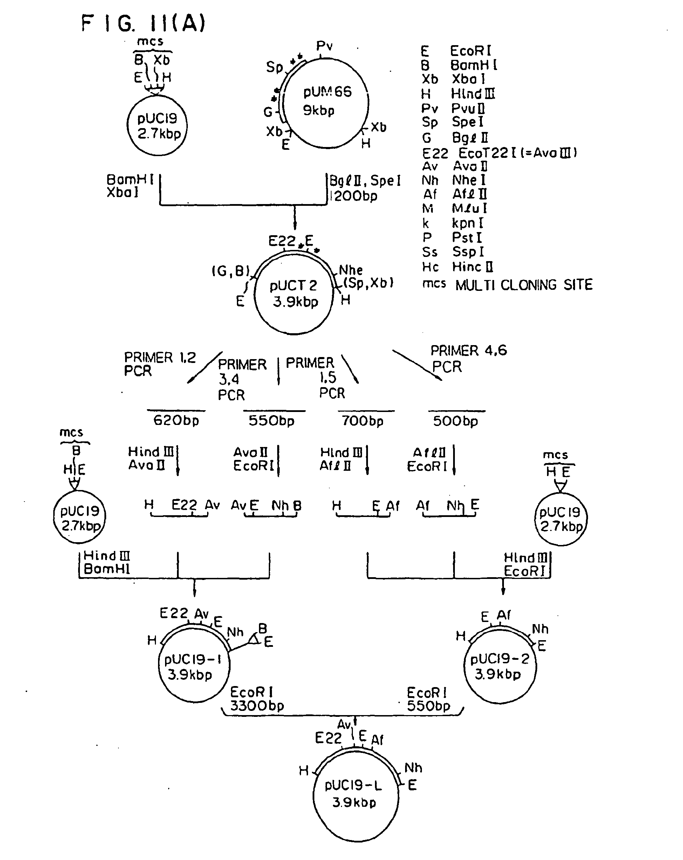

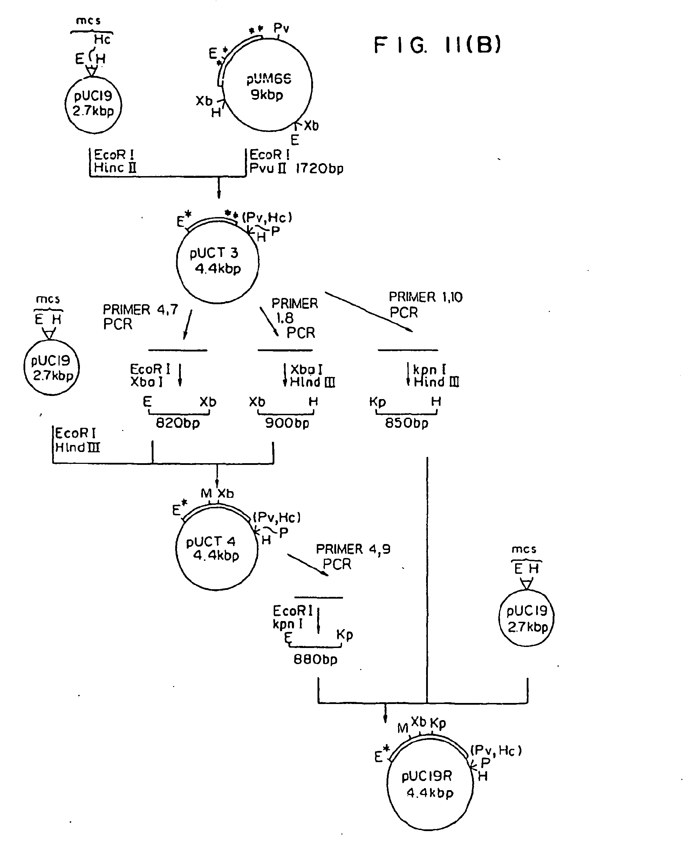

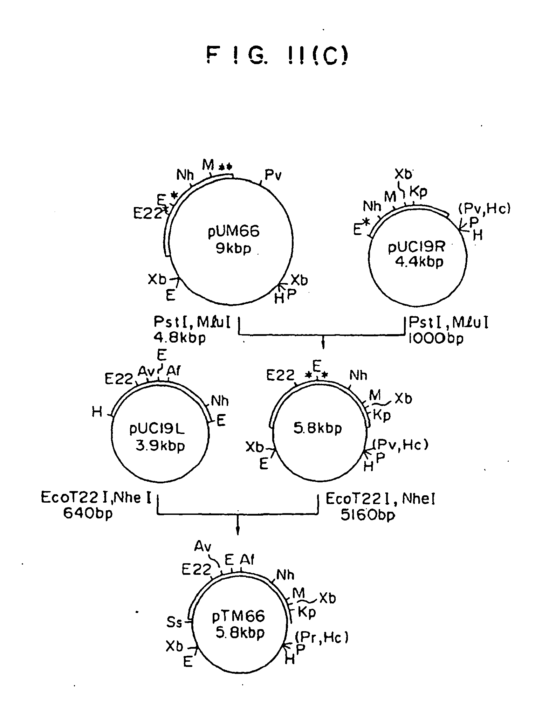

### (3) Construction of pTM66 modified (TGA + TGG) not to read TGA in ORF encoding TM-66 as translation termination codon (see Figs. 10 and 11 (A) through (C))

In order to modify the TGA codon in ORF of TM-66 to TGG codon, the change was made using polymerase chain reaction (PCR: Science, 230, 1350, 1354 (1985)) as in TM-67. DNA primers for PCR synthesized for the change are shown by SEQ ID NOS: 34-43.

Primers 1 to 10 corresponding to SEQ ID NOS: 34-43 using PCR are as follows.
Primer-1 5'-CAGGAAACAGCTATGAC-3' (M13 RV primer)

Primer-2 3'-GTTCTTCCTGGCAAACTTTA-5'
AvaII

Primer-3 5'-AAGAAGGACCGTTTGGAATG-3'
AvaII

Primer-4 5'-GTTTTCCCAGTCACGAC-3' (M13 primer)

Primer-5  3'-CAAAGTACCTAAATATCGAATTCACCT-5'

AflII

Primer-6 5'-ATAGCTTAAGTGGAACAAACACG-3'
AflII

Primer-7 3'-GGAACCAGATCTTGTTTCCC-5'
XbaI

Primer-8  5'-GGTCTAGAACAAAGGGATTGGACA-3'

XbaI

Primer-9  3'-CTACCTACCATGGTGATGAT-5'

KpnI

Primer-10 5'-GATGGTACCACTACTATTTCATGGACA-3'
KpnI

pUM66 was digested with B_{g}1II and SpeI and the fragment of about 1.2 kbp was recovered from 0.5% low melting agarose. The 1.2 kbp fragment was ligated with the digestion product of pUC19 with BamHI and XbaI to obtain pUCT2 (3.9 kbp). Next, using pUCT2 as a template and using Primer-1 and Primer-2, the fragment of 620 bp was amplified following conventional procedures for PCR and then recovered; after amplification of the fragment of about 550 bp using Primer-3 and Primer-4, the amplified fragment was recovered. Furthermore, the fragment of about 620 bp was digested with HindIII and AvaII; the fragment of 550 bp was digested with AvaII and BamHI. These fragments were ligated with the digestion product of pUC19 with HindIII and BamHI by ligase, respectively. The resulting plasmid was extracted and named pUC19-1 (3.9 kbp).

Next, using pUCT2 as a template and using Primer-4 and Primer-6, the fragment of about 500 bp was amplified following conventional procedures for PCR and then recovered; after amplification of the fragment of about 700 bp using Primer-1 and ,Primer-5, the amplified fragment was recovered. Furthermore, the fragment of about 500 bp was digested with AflII and EcoRI; the fragment of about 700 bp was digested with HindIII and AflII. These fragments were ligated with the digestion product of pUC19 with HindIII and AflII by ligase. The resulting plasmid was extracted and named pUC19-2 (about 3.9 kbp). Furthermore, pUC19-1 was digested with EcoRI and the digestion product was subjected to 0.6% low melting agarose gel electrophoresis to recover the fragment of about 3.3 kbp. pUC19-2 was also digested with EcoRI and the digestion product was subjected to 2.0% low melting agarose gel electrophoresis to recover the fragment of about 550 bp. This fragment was ligated with the about 3.3 kbp fragment derived from pUC19-1 described above using ligase to obtain plasmid pUC19L bearing the fragment in which two TGA codons at the 5' end in ORF of TM-66 have been changed to TGG.

In order to change two TGA codons at the 3' end of ORF of TM66 to TGG, firstly pUM66 was digested with EcoRI and PvuII and the fragment of about 1720 bp was recovered from 0.6% low melting agarose gel. The recovered fragment was ligated with the digestion product of pUC19 with EcoRI and HincII to obtain plasmid pUCT3 (about 4.4 kbp). Using pUCT3 as a template and using Primer-4 and Primer-7, the fragment of about 820 bp was amplified following conventional procedures for PCR and also the fragment of about 900 bp using Primer-8 and Primer-1 was amplified likewise, and the both fragments were then recovered, respectively. After this 820 bp fragment was digested with EcoRI and XbaI, the digestion product was ligated with the aforesaid about 900 bp fragment obtained by digestion with XbaI and HindIII and the digestion product of pUC19 with HindIII and EcoRI, using ligase to obtain plasmid pUCT4 (about 4.4 kbp). Next, using pOCT-4 as a template and also using Primers-4 and Primer-9, the fragment of about 880 bp was amplified following conventional procedures for PCR and also the fragment of about 900 bp using pUCT3 as a primer and using Primer-I and Primer-10 was amplified likewise following the conventional procedures for PCR; and the fragments were then recovered, respectively. After this 880 bp fragment was digested with EcoRI and KpnI, the digestion product was ligated with the aforesaid about 850 bp fragment obtained by digestion with HindIII and KpnI and the digestion product of pUC19 with EcoRI and HindIII, using ligase to obtain plasmid pUC19R.

In order to obtain plasmid in which TGA codons in ORF of TM-66 are all changed to TGG, pUM66 was digested with MluI and PvuII and the fragment of about 4.8 kbp was then recovered from 0.6% low melting agarose gel. The recovered fragment was ligated with the about 1.0 kbp fragment obtained by the digestion of pUC19R with MluI and PstI to obtain plasmid. This plasmid was further digested with EcoT22I and NheI. The resulting fragment of about 5.2 kbp was ligated with the fragment of about 640 bp obtained by the digestion of pUC19L with EcoT22I and NheI, using ligase to obtain plasmid bearing the full length of ORF in which TGA codons in ORF of TM-66 were all changed to TGG. This plasmid was named pTM66 (about 5.8 kbp).

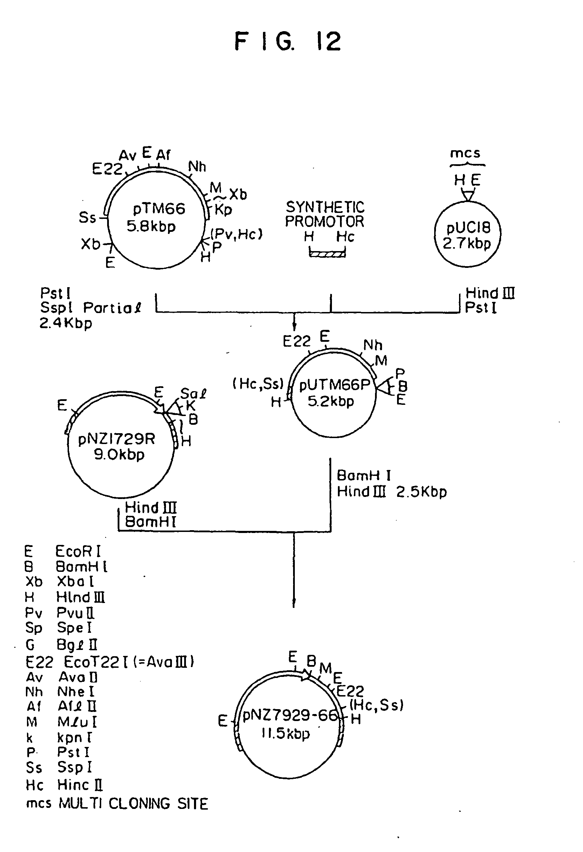

### (4) Construction of pNZ7929-66 (Fig. 12)

After pTM66 was digested with PstI, the digestion product was partially digested with SspI to recover the fragment of about 2.4 kbp. Three of the about 2.4 kbp fragment, the fragment obtained by the digestion of the synthetic promoter in Reference Example with HindIII and HincII and the fragment obtained by the digestion of pUC18 with HindIII and PstI were ligated using ligase to obtain pUTM66P (about 5.2 kbp). Next, pUTM66P was digested with HindIII and BamHI and the digestion product was recovered from low melting agarose gel. This fragment (about 2.5 kbp) was ligated with the fragment obtained by the digestion of pNZ1729R with HindIII and BamHI, using ligase to obtain the desired plasmid pNZ7929-66 (about 11.5 kbp).

### (5) Construction of fNZ7929-66 and purification

The procedures similar to Example 1 (3) were repeated using pNZ7929-66 obtained in (4) described above to obtain fNZ7929-66.

### Example 8

### Expression of TM-67 and TM-66 polypeptides in cells infected with fNZ7929-67 and fNZ7929-66

In order to examine that fNZ7929-67 and fNZ7929XM66 express the TM-67 and TM-66 polypeptides in infected cells, the immuno-fluorescence antibody method was carried out. fNZ7929-67 and fNZ7929-66 were infected to CEF, respectively and cultured at 37°C until plaques appeared. Thereafter the medium was fixed with cold acetone. Using Mycoplasma gallisepticum S6-immunized chicken serum or Mycoplasma gallisepticum-infected chicken serum as a primary antibody, the medium was diluted to 100- to 1000-fold and the dilution was reacted. These culture cells were further reacted with fluorescence (FITC)-bound anti-chick immunoglobulin. After washing out the non-specific reaction portion, microscopic observation was made under fluorescence-excited wavelength. The reactivity is shown in Table 3.

**Table 3. Reactivity of recombinant virus-infected CEP to various antisera**

| Reactivity to primary antibody Infected | | | |
|---|---|---|---|
| Infected virus | | | |
| | anti-S6 | S6 | SPF |
| fNZ7929-67 | +++ | +++ | - |
| fNZ7929-66 | +++ | +++ | - |
| fNZ2929XM1 | ++ | ++ | - |
| NP | - | - | - |

| | | | |
|---|---|---|---|
| +++ : strongly reacted over the entire surface ++ : strongly reacted + : reacted ± : weakly reacted - : not reacted | | | |

The results reveal that fNZ7929-67, fNZ7929-66 and fNZ2929XM1 which are the recombinant viruses were reactive with anti-S6 and S6 infection that are reactive with the infected cells alone.

### Example 9

### Activity of inhibiting the growth of an induced antibody of recombinant FPV-inoculated chick

After fNZ7929-67 and fNZ7929-66 were cultured in CEF at 37°C for 48 hours, the procedure of freezing and thawing was repeated twice to recover the cell suspension. The cell suspension was adjusted to have a virus titer of 10⁶ pfu/ml and then inoculated through a stab needle to SPF chicken (Line M, Nippon Seibutsu Kagaku Kenkyusho) of 7 days old at the right wing web in a dose of 10 µl. After the inoculation, generation of the pock was observed. Two weeks after the inoculation, sera were collected.

On the other hand, Mycoplasma gallisepticum S6 was inoculated on PPLO liquid medium (modified Chanock's medium) in a 10% concentration. After incubation at 37°C for 3 days, the cell mass was removed through a membrane filter of 0.45 µm. The filtrate was diluted with PPLO liquid medium in a cell count of 103 CFU/ml and the resulting dilution was provided as the cell solution for determination of activity.

The cell solution was put in a polypropylene tube by 400 µl each and 100 µl each of standard chick serum, TMG-1 immunized serum (Japanese Patent Application Laid-Open No. 2-111795) and various sera were added thereto, respectively. By incubation at 37°C for 2 to 5 days, growth inhibition test was conducted.

On Days 0, 1, 2, 3 and 4 after the incubation, 10 µl each was collected from the culture broth for Mycoplasma gallisepticum (hereinafter abbreviated as MG) growth inhibition test and spread over PPLO agar medium followed by incubation at 37°C for 7 days. The corresponding cell count in the culture broth was deduced from the number of colonies appeared. The results of measuring the cell count on Day 3 are shown in Table 4.

**Table 4**

| Sample | Cell Count on Day 3 |
|---|---|
| SPF chicken sera | 1.3 x 10⁸ |
| anti-TTMG-1 chicken sera | 1.8 x 10⁵ |
| fNZ2929XMI-inoculated chicken sera | 4.5 x 10⁵ |
| fNZ7929-67-inoculated chicken sera | 2.8 x 10⁴ |
| fNZ7929-66-inoculated chicken sera | 3.2 x 10⁴ |

In the culture broth of the medium in which SPF chick sera or equine sera were supplemented, there was no difference in growth rate of MG and the cell count reached saturation on Day 3 of the incubation. In the culture broth in which fNZ7929-67- or fNZ7929-66-inoculated sera were added, the growth of MG was more effectively inhibited than the case of fNZ2929XMI or than the case of immunizing an antigen inducing an antibody for inhibiting the growth of MG as in anti-TTMG-1 chick sera. This fact indicates that TM67 polypeptide and TM66 polypeptide are antigens capable of inducing antibodies which can inhibit the growth of MG more effectively than in TTMG-1.

### Reference Example 10

### Obtaining of polypeptide DNA TM-16 in which Mycoplasma gallisepticum is expressed

### (1) Preparation of Mycoplasma gallisepticum genomic DNA

Mycoplasma gallisepticum S6 strain was incubated at 37°C for 3 to 5 days in liquid medium prepared by adding to 10 ml of PPLO broth basal medium 20% equine sera, 5% yeast extract, 1% glucose and a trace amount of Phenol Red as a pH indicator. As Mycoplasma gallisepticum grew, the pH of the culture medium decreased. At the time when the color of the pH indicator contained in the medium was changed from red to yellow, the incubation was terminated. After the culture medium was centrifuged at 8000G for 20 minutes, the cells were collected. The cells were suspended in PBS in a 1/10 volume of the medium. The suspension was again centrifuged at 10,000 rpm for 20 minutes and the cells were then collected. The collected cells were again suspended in 2.7 ml of PBS. After SDS was added to the suspension in a concentration of 1% and further 10 µg of RNase was added thereto, incubation was performed at 37°C for 30 minutes for lysis.

The lysate was extracted 3 times with an equal volume of phenol and then 3 times with ethyl ether. By ethanol precipitation, 200 µg of Mycoplasma gallisepticum genomic DNA was obtained.

### (2) Genomic Southern hybridization of Mycoplasma gallisepticum using M-16 DNA gene as a probe

After 1 µg of the Mycoplasma gallisepticum DNA obtained in (1) described above was digested with XbaI, the digestion product was subjected to 0.6% low melting agarose gel electrophoresis. After the electrophoresis, the gel was immersed in an alkaline denaturation solution (0.5 M NaOH, 1.5 M NaCl) for 10 minutes to denature DNA. The gel was then immersed in a neutralization solution (3 M sodium acetate, pH 5.5) for 10 minutes for neutralization and then transferred onto a nylon membrane in 6-fold SSC solution (0.7 M NaCl, 0.07 M sodium citrate, pH 7.5). After air-drying, the nylon membrane was baked at 80°C for 2 hours and 4-fold SET (0.6 M NaCl, 0.08 M Tris-HCl, 4 mM EDTA, pH 7.8)-10-fold Denhardt-0.1% SDS-0.1% Na₄P₂O₇-50 µg/ml of denatured salmon sperm DNA and pUM-16 (M-16 gene is contained in this plasmid; cf. Japanese Patent Application Laid-Open No. 2-111795) labelled in a conventional manner, was added thereto to perform hybridization at 68°C for 14 hours. The nylon membrane was overlaid on an X ray film. It was confirmed by autoradiography that hybridization occurred to the about 5.5 kbp fragment.

### (3) Cloning of XbaI-digested fragment of about 5.5 kbp to pUC-19 and colony hybridization

After 4 µg of the Mycoplasma gallisepticum DNA obtained in (1) described above was digested with XbaI, the digestion product was subjected to 0.6% low melting agarose gel electrophoresis to recover the fragment of about 5.5 kbp. The fragment was ligated with the digestion product of pUC-19 with XbaI using ligase. Competent Escherichia coli TG1 strain was transformed with the ligation product. The transformants were cultured at 37°C for 15 hours in LB agar medium supplemented with 0.003% of 5-bromo-4-chloro-3-indolyl-β-D-galactopyranoside, 0.03 mM of isopropylthio-β-D-galactopyranoside and 40 µg/ml of ampicillin. White colonies grown on the medium were transferred onto a nylon membrane and hybridization was carried out in a manner similar to (2) described above. It was confirmed by autoradiography that cloning was effected and this plasmid was named pUM16.

### (8) Sequence analysis of pUM-16 insert DNA

The sequence of about 5.5 kbp fragment inserted into pUM-16 prepared in (3) described above was analyzed by the dideoxy method by Sanger et al. (Proc. Natl. Acad. Sci. USA, 74, 5463 (1977)).

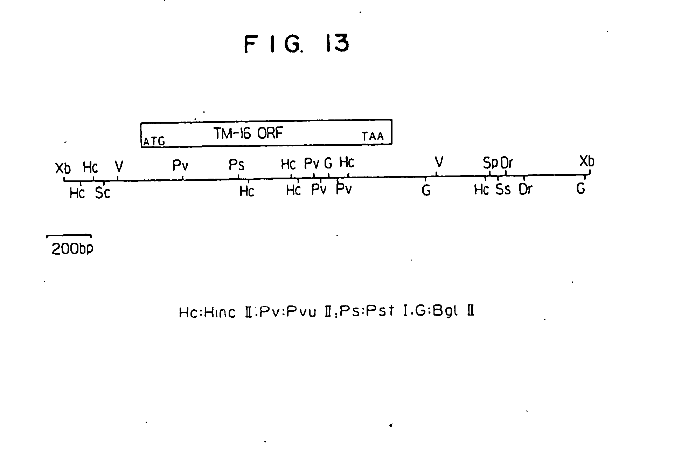

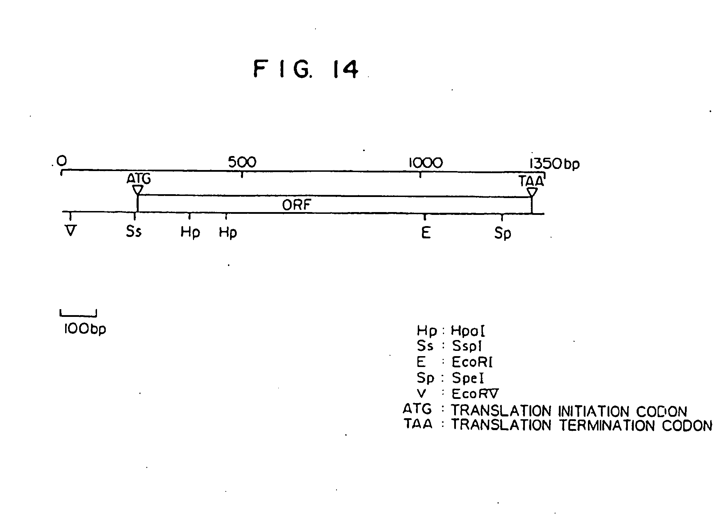

The restriction enzyme map of this fragment is shown in Fig. 13. The restriction enzyme map of the open reading frame present in this fragment is also shown in Fig. 14 and the nucleotide sequence of this ORF and the amino acid sequence deduced therefrom are shown by SEQ ID NO: 15. The polypeptide deduced from this ORP was named TM-16 polypeptide.

Hereinafter the sequences employed are described as sequence listing. The sequences used for the primers are described basically from the 3' end. However, the primers depicted from the 5' end in the body of the specification are described from the 5' end to conform to the description.

The following numbered paragraphs (paras.) contain statements of broad combinations of technical features herein disclosed:-
1. A recombinant Avipox virus inserted with DNA encoding a polypeptide showing antigenicity to Mycoplasma gallisepticum.
2. A recombinant Avipox virus according to para. 1, wherein DNA encoding a signal membrane anchor of type II external membrane protein which infects to fowl is inserted at the terminus of DNA encoding a polypeptide showing an antigenicity to Mycoplasma gallisepticum.
3. A recombinant Avipox virus according to para. 2, wherein said DNA encoding a signal membrane anchor is DNA encoding a signal membrane anchor of Newcastle disease virus.
4. A recombinant Avipox virus according to para. 1, 2 or 3, wherein said inserted DNA encoding a polypeptide showing an antigenicity is DNA encoding a polypeptide showing an antigenicity which is reactive with Mycoplasma gallisepticum-immunized sera or Mycoplasma gallisepticum-infected sera and is substantially pure, its nucleotide sequence being shown by SEQ ID NO: 1, SEQ ID NO: 14, SEQ ID NO: 15, SEQ ID NO: 16 or SEQ ID NO: 27.
5. A recombinant Avipox virus according to para. 1 or 2, wherein said DNA has a nucleotide sequence shown by SEQ ID NO: 14 or SEQ ID NO: 15, or a nucleotide sequence having the function substantially equivalent thereto.
6. A recombinant live vaccine for poultry Mycoplasma gallisepticum infection comprising as an effective ingredient a recombinant Avipox virus according to para. 1, 2, 3, 4 or 5.
7. A substantially pure antigenic protein which is reactive with Mycoplasma gallisepticum-immunized sera or Mycoplasma gallisepticum-infected sera and encoded by a gene derived from Mycoplasma gallisepticum having a restriction enzyme map shown in Fig. 1 ,and a modified antigenic protein which may be modified so long as it shows an antigenicity equivalent thereto.
8. A gene encoding an antigenic protein according to para. 7.
9. A substantially pure antigenic protein which is reactive with Mycoplasma-immunized sera or Mycoplasma-infected sera and encoded by a gene derived from Mycoplasma gallisepticum having a restriction enzyme map shown in Fig. 7, and a modified antigenic protein which may be modified so long as it shows an antigenicity equivalent thereto.
10. A gene encoding an antigenic protein according to para. 9.
11. A substantially pure antigenic protein which is reactive with Mycoplasma gallisepticum-immunized sera or Mycoplasma gallisepticum-infected sera and encoded by a gene derived from Mycoplasma gallisepticum having a restriction enzyme map shown in Fig. 8, and a modified antigenic protein which may be modified so long as it shows an antigenicity equivalent thereto.
12. A gene encoding an antigenic protein according to para. 11.
13. A substantially pure antigenic protein which is reactive with Mycoplasma-immunized sera or Mycoplasma-infected sera and encoded by a gene derived from Mycoplasma gallisepticum having a restriction enzyme map shown in Fig. 10 and a modified antigenic protein which may be modified so long as it shows an antigenicity equivalent thereto.
14. A gene encoding an antigenic protein according to para. 13.
15. A fused protein comprising a polypeptide showing an antigenicity of Mycoplasma gallisepticum ligated at the N-terminus thereof with a signal membrane anchor of type II external membrane protein which infects to fowl.
16. A fused protein according to para. 11, wherein said signal membrane anchor is a signal membrane anchor of hemagglutinin neuraminidase of Newcastle disease virus.
17. A hybrid DNA encoding a fused protein according to para. 15.
18. A component vaccine comprising as an effective ingredient a protein according to para. 7, 9, 11, 13, 15 or 16.

### SEQUENCE LISTING

(1) General information:
   (i) Applicant: USA
      KATSUHIKO NAKANO
      SHUJI SAITO
      SETSUKO OKAWA
      YOSHIHIKO SHIONO
      KOICHI IRITANI
      SHIGEMI AOYAMA
      KIYOTO TAKAHASHI
      SAKIKO SAEKI
      IKUROU OSAWA
      HIRONO FUNATO
      Designated countries other than USA
      NIPPON ZEON CO., LTD.
      SHIONOGI PHARMACEUTICAL CO., LTD.
   (ii) Title of Invention:
      NEW POLYPEPTIDE, DNA ENCODING THE POLYPEPTIDE,
      RECOMBINANT VECTOR BEARING THE DNA AND
      RECOMBINANT VIRUS UTILIZING THE RECOMBINANT
      VECTOR AS WELL AS USE THEREOF
   (iii) Number of sequences: 43
(2) Information for SEQ ID No. 1
   (i) Sequence characteristics:
      (A) Length of sequence: 2369
      (B) Type of sequence: amino acid
      (C) Number of strand: double strand
      (D) Topology: linear
      (E) Kind of sequence: DNA
   (xi) Sequence description: SEQ ID NO: 1 
GTCTGCGGTT GGTTTGATCA GCGAAAATAA ACCCGATTTA TTACTTACTG AACTTTATAT 60
ATTCTTTAGA TAATAATAGA CGTGGTGAAC GTAAGTTATT GGCTTAACTT TAAGTGAAAA 120
GAAAAAACAT TTTAAAGTTT GTTAGTTTAT TAGGTATTGT TTCGTTTGTA ATG TTA 176
Met Leu

GCA GCT GCT AGT TGT ACT TCA GCA GCT ACA CCA ACT CCA AAC CCT GAA 224
Ala Ala Ala Ser Cys Thr Ser Ala Ala Thr Pro Thr Pro Asn Pro Glu
        5                   10                  15
CCA AAA CCA ACT CCA AAC CCT GAA CCA AAA CCA GAT CCA ATG CCA AAC 272
Pro Lys Pro Thr Pro Asn Pro Glu Pro Lys Pro Asp Pro Met Pro Asn
    20                  25                  30
CCT CCT AGT GGT GGT AAC ATG AAT GGT GGA AAC ACC AAC CCA AGT GAT 320
Pro Pro Ser Gly Gly Asn Met Asn Gly Gly Asn Thr Asn Pro Ser Asp
35                  40                  45                  50
GGC CAA GGC ATG ATG AAT GCA GCT GCT AAA GAA TTA GCA GAC GCA AAA 368
Gly Gln Gly Met Met Asn Ala Ala Ala Lys Glu Leu Ala Asp Ala Lys
                55                  60                  65
GCT GCT TTA ACT ACT TTG ATT AAT GGT GAA ACT GCA AAT CTT GCG TCA 416
Ala Ala Leu Thr Thr Leu Ile Asn Gly Glu Thr Ala Asn Leu Ala Ser
            70                  75                  80
TAT GAA GAC TAT GCT AAG ATC AAA AGT GAA TTA ACA TCA GCG TAT GAA 464
Tyr Glu Asp Tyr Ala Lys Ile Lys Ser Glu Leu Thr Ser Ala Tyr Glu
        85                  90                  95
ACA GCT AAA GCA GTT TCA GCT AAA ACT GGT GCA ACT CTA AAT GAG GTT 512
Thr Ala Lys Ala Val Ser Ala Lys Thr Gly Ala Thr Leu Asn Glu Val
    100                 105                 110
 
AAT GAG GCA AAA ACT ACA TTA GAT GCT GCT ATT AAA AAA GCT GCT AGT 560
Asn Glu Ala Lys Thr Thr Leu Asp Ala Ala Ile Lys Lys Ala Ala Ser
115                 120                 125                 130
GCT AAG AAT GAT TTT GAT GCA CAG CAC GGG TCA CTA GTG GAA GCA TAT 608
Ala Lys Asn Asp Phe Asp Ala Gln His Gly Ser Leu Val Glu Ala Tyr
                135                 140                 145
AAC AAT CTA AAA GAA ACG TTA [?]AA GAA GAA AAA ACT AAT TTA GAT TCT 656
Asn Asn Leu Lys Glu Thr Leu Lys Glu Glu Lys Thr Asn Leu Asp Ser
            150                 155                 160
CTT GCA AAC GAA AAT TAT GCA GCA ATC AGA ACT AAT CTT AAT AGT TTA 704
Leu Ala Asn Glu Asn Tyr Ala Ala Ile Arg Thr Asn Leu Asn Ser Leu
        165                 170                 175
TAT GAA AAA GCC AAT ACT ATT GTT ACA GCT ACT TTA GAC CCT GCT ACT 752
Tyr Glu Lys Ala Asn Thr Ile Val Thr Ala Thr Leu Asp Pro Ala Thr
    180                 185                 190
GGA AAT ATT CCT GAA GTT ATG AGT GTA ACA CAA GCT AAT CAA GAT ATT 800
Gly Asn Ile Pro Glu Val Met Ser Val Thr Gln Ala Asn Gln Asp Ile
195                 200                 205                 210
ACT AAT GCA ACT TCA AGA CTA ATA GCT TGA AAA CAA AAT GCT GAT AAT 848
Thr Asn Ala Thr Ser Arg Leu Ile Ala Trp Lys Gln Asn Ala Asp Asn
                215                 220                 225
TTA GCT AAC AGT TTT ATC AAA CAG TCT TTA GTT AAA AAT AAT TTG ACT 896
Leu Ala Asn Ser Phe Ile Lys Gln Ser Leu Val Lys Asn Asn Leu Thr
            230                 235                 240
AGA GTT GAT GTA GCA AAT AAT CAG GAG CAA CCA GCA AAT TAC AGT TTT 944
Arg Val Asp Val Ala Asn Asn Gln Glu Gln Pro Ala Asn Tyr Ser Phe
        245                 250                 255
GTT GGT TTT AGT GTT AAT GTT GAT ACT CCT AAC TGA AAT TTT GCG CAA 992
Val Gly Phe Ser Val Asn Val Asp Thr Pro Asn Trp Asn Phe Ala Gln
    260                 265                 270
 
AGA AAA GTT TGG GCC TCT GAA AAT ACT CCT TTA GCA ACT ACA CCA GCT 1040
Arg Lys Val Trp Ala Ser Glu Asn Thr Pro Leu Ala Thr Thr Pro Ala
275                 280                 285                 290
GAA GAT GCA ACA CAA CAA GCT GCA TCC TTA ACA GAT GTT TCA TGA ATC 1088
Glu Asp Ala Thr Gln Gln Ala Ala Ser Leu Thr Asp Val Ser Trp Ile
                295                 300                 305
TAT AGT TTA AAT GGT GCT GAA GCT AAA TAC ACA TTA AGC TTT CGT TAC 1136
Tyr Ser Leu Asn Gly Ala Glu Ala Lys Tyr Thr Leu Ser Phe Arg Tyr
            310                 315                 320
TTT GGA GCT GAA AAA ACA GCT TAC TTA TAT TTC CCT TAT AAA TTA GTT 1184
Phe Gly Ala Glu Lys Thr Ala Tyr Leu Tyr Phe Pro Tyr Lys Leu Val
        325                 330                 335
AAA ACT AGT GAT AAT GTT GGT TTA CAA TAT AAG TTA AAT GGT GGT GAT 1232
Lys Thr Ser Asp Asn Val Gly Leu Gln Tyr Lys Leu Asn Gly Gly Asp
    340                 345                 350
ACT AAA CAA ATT AAC TTT GTA CAA ACT CCA GCT TCT GGT TCA AGT GAT 1280
Thr Lys Gln Ile Asn Phe Val Gln Thr Pro Ala Ser Gly Ser Ser Asp
355                 360                 365                 370
GTT GCT GCT AAT GAA GAA GAA ACT ATG GCT AGT CCT GCT GAA ATG CAG 1328
Val Ala Ala Asn Glu Glu Glu Thr Met Ala Ser Pro Ala Glu Met Gln
                375                 380                 385
TCA GCA CCA ACT GTT Asp Asp Ile Lys Ile Ala Lys Val Ala Leu Ser 1376
Ser Ala Pro Thr Val GAC GAT ATT AAG ATT GCT AAA GTC GCT TTA TCT
390 395 400
AAT CTA AAA TTC AAT TCA AAC ACA ATT GAA TTT AGT GTC CCT ACA GGT 1424
Asn Leu Lys Phe Asn Ser Asn Thr Ile Glu Phe Ser Val Pro Thr Gly
405 410 415
AAA GCA GCT CCT ATG ATT GGA AAT ATG TAT TTA ACT TCA TCT AAT TCG 1472
Lys Ala Ala Pro Met Ile Gly Asn Met Tyr Leu Thr Ser Ser Asn Ser
420 425 430
 
GAA GTT AAT AAA AAC AAA ATT TAT GAT GAT CTA TTC GGC AAC AGC TTT 1520
Glu Val Asn Lys Asn Lys Ile Tyr Asp Asp Leu Phe Gly Asn Ser Phe
435 440 445 450
AAT AAT GAA AAT AAT CCA ACC GCG GTT ACT GTT GAC CTA TTA AAA GGT 1568
Asn Asn Glu Asn Asn Pro Thr Ala Val Thr Val Asp Leu Leu Lys Gly
455 460 465
TAT AGT CTT GCT GCT AGT TAC AGT ATA TAT GTT CGC CAA TTC AAT GAT 1616
Tyr Ser Leu Ala Ala Ser Tyr Ser Ile Tyr Val Arg Gln Phe Asn Asp
470 475 480
TTA AAT ATT CAA AAT GGC ACT GAT ATG GCA AGA TCT CGA ACA GTA TAC 1664
Leu Asn Ile Gln Asn Gly Thr Asp Met Ala Arg Ser Arg Thr Val Tyr
485 490 495
TTA GTT GGG TTA ATT GGT AGT AAT GCA AGT AGA TCA ATT AGG AAC CTA 1712
Leu Val Gly Leu Ile Gly Ser Asn Ala Ser Arg Ser Ile Arg Asn Leu
500 505 510
TCA AAT GTA AGA ACT TCT CCT AAC ACG GTT AGT ACC AAT AGA ACA TTT 1760
Ser Asn Val Arg Thr Ser Pro Asn Thr Val Ser Thr Asn Arg Thr Phe
515 520 525 530
ACA ATA TAT GTA AAT GCT CCA AAG TCA GGT GAT TAT TAT CTA AGT GGT 1808
Thr Ile Tyr Val Asn Ala Pro Lys Ser Gly Asp Tyr Tyr Leu Ser Gly
535 540 545
TCG TAT CTT ACA AAT CAA AAT ACA AAT ATT AAA TTC TTA AAT AGC AGC 1856
Ser Tyr Leu Thr Asn Gln Asn Arg Asn Ile Lys Phe Leu Asn Ser Ser
550 555 560
TCT GAT CAG ACT AGT AGT AAT TCT CTA ACA CTA AAT GTT AAG GCT CAA 1904
Ser Asp Gln Thr Ser Ser Asn Ser Leu Thr Leu Asn Val Lys Ala Gln
565 570 575
ACA AAT TGA GAG ACT TTA GGA AAT TTC GAT ACA TCT AAT AAT ACG AAT 1952
Thr Asn Trp Glu Thr Leu Gly Asn Phe Asp Thr Ser Asn Asn Thr Asn
580 585 590
 
ATT GTT ACT AAT AGT GGA TCA AGC ACA ACA ACA GGC CGG ACT TTA AAT 2000
Ile Val Thr Asn Ser Gly Ser Ser Thr Thr Thr Gly Arg Thr Leu Asn
595 600 605 610
TTA AAA CAA GGA TTA AAC AAA ATT GTT ATC AGT GGA GTA GGT AAT GGT 2048
Leu Lys Gln Gly Leu Asn Lys Ile Val Ile Ser Gly Val Gly Asn Gly
615 620 625
AAT ACT CCT TTC ATA GGT AAC TTA ACA TTT ACT TTG ATG GAT AAA ACA 2096
Asn Thr Pro Phe Ile Gly Asn Leu Thr Phe Thr Leu Met Asp Lys Thr
630 635 640
GCT AGT CCT GTA GTT GAT GAC ACT ATT TTA GAA GGA TCT ATA GAA GCT 2144
Ala Ser Pro Val Val Asp Asp Thr Ile Leu Glu Gly Ser Ile Glu Ala
645 650 655
GGT TCA AAA TAA AAAATTATGT TTTTTTAAAT CTTTTTTCAA GGATCATGTT 2196
Gly Ser Lys ***
660
TCTGTTTAAA CGCTAAGTTA GTTAGATAAT AAAATAAAAG TTATTTGTTT TACTCCATGT 2256
AATATGGCAT GAAATCTCAA TCAAACTTCA GATTTCATGT TTTTTTTATT AAGGAAGCAA 2316
ATATGAGATA CTAGCAGCCT TTTGTCTACT ATACTTATGA TCGAACTAGA TCT 2369
(2) Information for SEQ ID Neo. 2
   (i) Sequence characteristics:
      (A) Length of sequence: 48
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 2
      GATCTTCCAT TTTAGGATCT ATATTATTTT TTCAACGATC CGAGCTCG 48
(2) Information for SEQ ID NO. 3
   (i) Sequence characteristics:
      (A) Length of sequence: 48
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 3
      CATCTTCCAT TTTAGGATCT ATATTATTTT TTCAACGATC CCAGCTCG 48
(2) Information for SEQ ID NO. 4
   (i) Sequence characteristics:
      (A) Length of sequence: 55
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 4
      AGCTTTTTTT TTTTTTTTTT TTTGGCATAT AAATAATAAA TACAATAATT AATTA 55
(2) Information for SEQ ID No. 5
   (i) Sequence characteristics:
      (A) Length of sequence: 55
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 5
      CGCGTAATTA ATTATTGTAT TTATTATTTA TATGCCAAAA AAAAAAAAAA AAAAA 55
(2) Information for SEQ ID No. 6
   (i) Sequence characteristics:
      (A) Length of sequence: 40
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 6
      CGCGTAAAAA TTGAAAAACT ATTCTAATTT ATTGCACTCG 40
(2) Information for SEQ ID No. 7
   (i) Sequence characteristics:
      (A) Length of sequence: 40
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 7
      GATCCGAGTG CAATAAATTA.GAATAGTTTT TCAATTTTTA 40
(2) Information for SEQ ID No. 8
   (i) Sequence characteristics:
      (A) Length of sequence: 42
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 8
      GATCCCCGGG CGAGCTCGCT AGCGGGCCCG CATGCGGTAC CG 42
(2) Information for SEQ ID No. 9
   (i) Sequence characteristics:
      (A) Length of sequence: 42
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 9
      TCGACGGATC CGCATGCGGG CCCGCTAGCG AGCTCGCCCG GG 42
(2) Information for SEQ ID NO. 10
   (i) Sequence characteristics:
      (A) Length of sequence: 39
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 10
      TCGACCCGGT ACATTTTTAT AAAAATGTAC CCGGGGATC 39
(2) Information for SEQ ID NO. 11
   (i) Sequence characteristics:
      (A) Length of sequence: 35
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 11
      GATCCCCGGG TACATTTTTA TAAAAATGTA CCGGG 35
(2) Information for SEQ ID NO. 12
   (i) Sequence characteristics:
      (A) Length of sequence: 14
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 12
      ATTTTTATAA AAAT 14
(2) Information for SEQ ID NO. 13
   (i) Sequence characteristics:
      (A) Length of sequence: 66
      (B) Type of sequence: amino acid
      (C) Number of strand: single
      (D) Topology: linear
      (E) Kind of sequence: DNA
   (xi) Indication of sequence: SEQ ID NO: 13 
ATC GCG ATC CTA CTT TTA ACA GTA GTG ACC TTA GCC ATC TCT GCA GCC 48
Ile Ala Ile Leu Leu Leu Thr Val Val Thr Leu Ala Ile Ser Ala Ala
                5                   10                  15
GCC CTT GCA TAT AGT ATG 66
Ala Leu Ala Tyr Ser Met
            20
(2) Information for SEQ ID NO. 14
   (i) Sequence characteristics:
      (A) Length of sequence: 1387
      (B) Type of sequence: amino acid
      (C) Number of strand: double
      (D) Topology: linear
      (E) Kind of sequence: DNA
   (xi) Indication of sequence: SEQ ID NO: 14 
AAAAACATCA GATTGTTAAT CTGATATCTT TGCTTAAAAA AACACAAAAT CTTCTAACAA 60
AATCCTAAAT AAATAAGCCG TTAAATTAAC TAAAAAATTA AAAAAATGCT TTTTCTTATC 120
AACCAAAATT CTCTAGTAAT AAACGCTTAT TTATTTTTAT TTTTAGTCAT CTTTTAAGAT 180
ATAAATATAT CTTAATATTC T ATG AAT AAG AAA AGA ATC ATC TTA AAG ACT 231
Met Asn Lys Lys Arg Ile Ile Leu Lys Thr
                5                   10

ATT AGT TTG TTA GGT ACA ACA TCC TTT CTT AGC ATT GGG ATT TCT AGC 279
Ile Ser Leu Leu Gly Thr Thr Ser Phe Leu Ser Ile Gly Ile Ser Ser
                15                  20                  25

TGT ATG TCT ATT ACT AAA AAA GAC GCA AAC CCA AAT AAT GGC CAA ACC 375
Cys Met Ser Ile Thr Lys Lys Asp Ala Asn Pro Asn Asn Gly Gln Thr
            30                  35                  40

CAA TTA CAA GCA GCG CGA ATG GAG TTA ACT GAT CTA ATC AAT GCT AAA 327
Gln Leu Gln Ala Ala Arg Met Glu Leu Thr Asp Leu Ile Asn Ala Lys
        45                  50                  55

GCA AGG ACA TTA GCT TCA CTA CAA GAC TAT GCT AAG ATT GAA GCT AGT 423
Ala Arg Thr Leu Ala Ser Leu Gln Asp Tyr Ala Lys Ile Glu Ala Ser
    60                  65                  70

TTA TCA TCT GCT TAT AGT GAA GCT GAA ACA GTT AAC AAT AAC CTT AAT 471
Leu Ser Ser Ala Tyr Ser Glu Ala Glu Thr Val Asn Asn Asn Leu Asn
75                  80                  85                  90

GCA ACA CTA GAA CAA CTA AAA ATG GCT AAA ACT AAT TTA GAA TCA GCC 519
Ala Thr Leu Glu Gln Leu Lys Met Ala Lys Thr Asn Leu Glu Ser Ala
                95                  100                 105

ATC AAC CAA GCT AAT ACG GAT AAA ACG ACT TTT GAT AAT GAA CAT CCA 567
Ile Asn Gln Ala Asn Thr Asp Lys Thr Thr Phe Asp Asn Glu His Pro
            110                 115                 120
 
AAT TTA GTT GAA GCA TAC AAA GCA CTA AAA ACC ACT TTA GAA CAA CGT 615
Asn Leu Val Glu Ala Tyr Lys Ala Leu Lys Thr Thr Leu Glu Gln Arg
        125                 130                 135

GCT ACT AAC CTT GAA GGT TTA GCT TCA ACT GCT TAT AAT CAG ATT CGT 663
Ala Thr Asn Leu Glu Gly Leu Ala Ser Thr Ala Tyr Asn Gln Ile Arg
    140                 145                 150

AAT AAT TTA GTG GAT CTA TAC AAT AAT GCT AGT AGT TTA ATA ACT AAA 711
Asn Asn Leu Val Asp Leu Tyr Asn Asn Ala Ser Ser Leu Ile Thr Lys
155                 160                 165                 170
ACA CTA GAT CCA CTA AAT GGG GGA ATG CTT TTA GAT TCT AAT GAG ATT 759
Thr Leu Asp Pro Leu Asn Gly Gly Met Leu Leu Asp Ser Asn Glu Ile
                175                 180                 185

ACT ACA GTT AAT CGG AAT ATT AAT AAT ACG TTA TCA ACT ATT AAT GAA 807
Thr Thr Val Asn Arg Asn Ile Asn Asn Thr Leu Ser Thr Ile Asn Glu
            190                 195                 200

CAA AAG ACT AAT GCT GAT GCA TTA TCT AAT AGT TTT ATT AAA AAA GTG 855
Gln Lys Thr Asn Ala Asp Ala Leu Ser Asn Ser Phe Ile Lys Lys Val
        205                 210                 215

ATT CAA AAT AAT GAA CAA AGT TTT GTA GGG ACT TTT ACA AAC GCT AAT 903
Ile Gln Asn Asn Glu Gln Ser Phe Val Gly Thr Phe Thr Asn Ala Asn
    220                 225                 230

GTT CAA CCT TCA AAC TAC AGT TTT GTT GCT TTT AGT GCT GAT GTA ACA 951
Val Gln Pro Ser Asn Tyr Ser Phe Val Ala Phe Ser Ala Asp Val Thr
235                 240                 245                 250

CCC GTC AAT TAT AAA TAT GCA AGA AGG ACC GTT NNN AAT GGT GAT GAA 999
Pro Val Asn Tyr Lys Tyr Ala Arg Arg Thr Val Xaa Asn Gly Asp Glu
                255                 260                 265
 
AAT TTA GTT GAA GCA TAC AAA GCA CTA AAA ACC ACT TTA GAA CAA CGT 615
Asn Leu Val Glu Ala Tyr Lys Ala Leu Lys Thr Thr Leu Glu Gln Arg
125 130 135

GCT ACT AAC CTT GAA GGT TTA GCT TCA ACT GCT TAT AAT CAG ATT CGT 663
Ala Thr Asn Leu Glu Gly Leu Ala Ser Thr Ala Tyr Asn Gln Ile Arg
140 145 150

AAT AAT TTA GTG GAT CTA TAC AAT AAT GCT AGT AGT TTA ATA ACT AAA 711
Asn Asn Leu Val Asp Leu Tyr Asn Asn Ala Ser Ser Leu Ile Thr Lys
155 160 165 170
ACA CTA GAT CCA CTA AAT GGC GGA ATG CTT TTA GAT TCT AAT GAG ATT 759
Thr Leu Asp Pro Leu Asn Gly Gly Met Leu Leu Asp Ser Asn Glu Ile
175 180 185

ACT ACA GTT AAT CGG AAT ATT AAT AAT ACG TTA TCA ACT ATT AAT GAA 807
Thr Thr Val Asn Arg Asn Ile Asn Asn Thr Leu Ser Thr Ile Asn Glu
190 195 200

CAA AAG ACT AAT GCT GAT GCA TTA TCT AAT AGT TTT ATT AAA AAA GTG 855
Gln Lys Thr Asn Ala Asp Ala Leu Ser Asn Ser Phe Ile Lys Lys Val
205 210 215

ATT CAA AAT AAT GAA CAA AGT TTT GTA GGG ACT TTT ACA AAC GCT AAT 903
Ile Gln Asn Asn Glu Gln Ser Phe Val Gly Thr Phe Thr Asn Ala Asn
220 225 230

GTT CAA CCT TCA AAC TAC AGT TTT GTT GCT TTT AGT GCT GAT GTA ACA 951
Val Gln Pro Ser Asn Tyr Ser Phe Val Ala Phe Ser Ala Asp Val Thr
235 240 245 250

CCC GTC AAT TAT AAA TAT GCA AGA AGG ACC GTT NNN AAT GGT GAT GAA 999
Pro Val Asn Tyr Lys Tyr Ala Arg Arg Thr Val Xaa Asn Gly Asp Glu
255 260 265
 
CCT TCA AGT AGA ATT CTT GCA AAC ACG AAT AGT ATC ACA GAT GTT TCT 1047
Pro Ser Ser Arg Ile Leu Ala Asn Thr Asn Ser Ile Thr Asp Val Ser
270 275 280

Xaa ATT TAT AGT TTA GCT GGA ACA AAC ACG AAG TAC CAA TTT AGT TTT 1095
NNN Ile Tyr Ser Leu Ala Gly Thr Asn Thr Lys Tyr Gln Phe Ser Phe
285 290 295

AGC AAC TAT GGT CCA TCA ACT GGT TAT TTA TAT TTC CCT TAT AAG TTG 1143
Ser Asn Tyr Gly Pro Ser Thr Gly Tyr Leu Tyr Phe Pro Tyr Lys Leu
300 305 310

GTT AAA GCA GCT GAT GCT AAT AAC GTT GGA TTA CAA TAC AAA TTA AAT 1191
Val Lys Ala Ala Asp Ala Asn Asn Val Gly Leu Gln Tyr Lys Leu Asn
315 320 325 330

AAT GGA AAT GTT CAA CAA GTT GAG TTT GCC ACT TCA ACT AGT GCA AAT 1239
Asn Gly Asn Val Gln Gln Val Glu Phe Ala Thr Ser Thr Ser Ala Asn
335 340 345

AAT ACT ACA GCT AAT CCA ACT CAG CAG TTG ATG AGA TTA AAG TTG CTA 1287
Asn Thr Thr Ala Asn Pro Thr Gln Gln Leu Met Arg Leu Lys Leu Leu
350 355 360

AAA TCC TTT TAT CAG GTT TAA CATTTGGCCA AAACACAATC GAATTAAGTG 1338
Lys Ser Phe Tyr Gln Val ***
365

TTCCAACGGG TGAAGGAAAT ATGAATAAAG TTGCGCCAAT GATTGGCAA 1387
(2) Information for SEQ ID NO. 15
   (i) Sequence characteristics:
      (A) Length of sequence: 1945
      (B) Type of sequence: amino acid
      (C) Number of strand: double
      (D) Topology: linear
      (E) Kind of sequence: DNA
   (xi) Indication of sequence: SEQ ID NO: 15 
CGTACGTTTT AATCGCTATT GGGCTCTTAT TTTATTGTCA GCATTGCAC TAACAGCAGTT 60
ATACCAAGC CCAATTAACT CAGTAGAAGT TACACAG ATG ATG AAT GGT CAA GAA 114
Met Met Asn Gly Gln Glu
                5
GTC ACA ACA ACT AAA AAG ATT AGT ACG TTT GCC TTC TTA ATC AAC ATG 162
Val Thr Thr Thr Lys Lys Ile Ser Thr Phe Ala Phe Leu Ile Asn Met
            10                  15                  20
TTA CCA AAT TAC CAA CTA AGT ACA CTT GGT TAC TTA CAG ATT ACA GCA 210
Leu Pro Asn Tyr Gln Leu Ser Thr Leu Gly Tyr Leu Gln Ile Thr Ala
        25                  30                  35
GCT GCT GCT GGA CTT GTA GTA GGG ATT GTA TTA CTT GCA TTA GGC GCA 258
Ala Ala Ala Gly Leu Val Val Gly Ile Val Leu Leu Ala Leu Gly Ala
    40                  45                  50
ACA TTC TTT GTT AAA ACT AGA CGT AAA ACA AAT GAA ATG CTT GCT GCA 306
Thr Phe Phe Val Lys Thr Arg Arg Lys Thr Asn Glu Met Leu Ala Ala
55                  60                  65                  70
CTT CAA GAT GCT GAA GAA GAA GAA GTG GCA CAA GAA GAA CAA GCT GAA 354
Leu Gln Asp Ala Glu Glu Glu Glu Val Ala Gln Glu Glu Gln Ala Glu
                75                  80                  85
GAA AAT GTT GAA GTC ACT CCA ACT CAA CAA GCT GAA GTT AAG ACT GAA 402
Glu Asn Val Glu Val Thr Pro Thr Gln Gln Ala Glu Val Lys Thr Glu
            90                  95                  100
CAA TTA ATT GGC ACA CAA TTA GTA ACA ACT GAT GTA GCT AGC AAT CAA 450
Gln Leu Ile Gly Thr Gln Leu Val Thr Thr Asp Val Ala Ser Asn Gln
        105                 110                 115
GCT GCA GGT ACT GAA CAA GTT GAA GGT GAT TTA TTA CCT CCT AGT CAA 498
Ala Ala Gly Thr Glu Gln Val Glu Gly Asp Leu Leu Pro Pro Ser Gln
    120                 125                 130
 
CAA CCA ACG GAA ATG CGT CCA GCT CCT TCA CCA ATG GGT AGT CCT AAG 546
Gln Pro Thr Glu Met Arg Pro Ala Pro Ser Pro Met Gly Ser Pro Lys
135                 140                 145                 150
TTA TTA GGT CCA AAC CAA GCT GGT CAT CCA CAA CAC GGA CCA CGT CCG 594
Leu Leu Gly Pro Asn Gln Ala Gly His Pro Gln His Gly Pro Arg Pro
                155                 160                 165
ATG AAT GCT CAT CCA GGT CAA CCA CGT CCT CAA CAA GCT GGC CCA CGT 642
Met Asn Ala His Pro Gly Gln Pro Arg Pro Gln Gln Ala Gly Pro Arg
            170                 175                 180
CCA ATG GGA GCT GGT GGA TCT AAC CAA CCA AGA CCC ATG CCA AAT GGT 690
Pro Met Gly Ala Gly Gly Ser Asn Gln Pro Arg Pro Met Pro Asn Gly
        185                 190                 195
CCA CAA AAC CAA CAA GGT CCA AGA CCA ATG AAC CCT CAA GGC AAT CCT 738
Pro Gln Asn Gln Gln Gly Pro Arg Pro Met Asn Pro Gln Gly Asn Pro
    200                 205                 210
CGT CCT GGA CCA GCT GGC CCA CGA CCT AAC GGC CCA CAA AAT TCT CAA 786
Arg Pro Gly Pro Ala Gly Pro Arg Pro Asn Gly Pro Gln Asn Ser Gln
215                 220                 225                 230
CCA CGT CCT CAA CCA GCT GGC CCA CGT CCA ATG GGA GCT GGT AGA TCT 834
Pro Arg Pro Gln Pro Ala Gly Pro Arg Pro Met Gly Ala Gly Arg Ser
                235                 240                 245
AAC CAA CCA AGA CCA ATG CCA AAT GGT CCA CAA AAC CAA CAA GGT CCA 882
Asn Gln Pro Arg Pro Met Pro Asn Gly Pro Gln Asn Gln Gln Gly Pro
            250                 255                 260
AGA CCA ATG AAC CCT CAA GGC AAT CCT CGT CCT CAA CCA GCT GGT GTC 930
Arg Pro Met Asn Pro Gln Gly Asn Pro Arg Pro Gln Pro Ala Gly Val
        265                 270                 275
AGA CCT AAC AGC CCA CAA GCT AAC CAG CCA GGA CCA CGT CCA ACG CCA 978
Arg Pro Asn Ser Pro Gln Ala Asn Gln Pro Gly Pro Arg Pro Thr Pro
280 285 200
 
AAT AAT CCT CAA GGA CCA CGG CCA ATG GGT CCA AGA CCA AAT GGA GGA 1026
Asn Asn Pro Gln Gly Pro Arg Pro Met Gly Pro Arg Pro Asn Gly Gly
295                 300                 305                 310

CCA AAC CGA GCT TAATTAACCA ATAGATTAGC TCTAAATTTG AAAACAGTTC 1078
Pro Asn Arg Ala

ATTTCCTAGA AAATGAACTG TTTTTTTTAT TATTTGTAAG TAAATTTATT AATCAACCGC 1138
TTGTTTTGTT GAATAAAGAT AGATCACAAC ATCTTCTTGA TTTACATCTT TAATTTGCAT 1198
ATTATTGATC ATTAAAGGGA TCTTGATGAT CTGATACATC TTGTTATTCT CATAATCAAG 1258
ATAATTAAGA TGTGAAGCAC TAAAAGCAAA TAGCTCTTGT TCAGATTGGA TTAGTTCTTT 1318
AGCATTATTT AAGAACGACT GATCATCACT CAGTAATAAT AAGATCTGAT TCAAGTTTTT 1378
CATATCAGTT CCTACTTCTT GATTTAACAT CAATGTTTCA TAGCGTGATA ATAAGGATTT 1438
AAAACGGTGA ATGATTGATG TCGTTGCACT TTTCTCATCG TTGGTTTCAA CGTATTGAAA 1498
AGTGTTCATT AAGTTAATGT ATTCTTGCTG GTATTTCTTA TTAATCTGAT CAGGGTTATC 1558
TGAATAGATT AAGATGTTCT TATTAGTTTG ATCAACAATA ACCATCGTTG CTTTCATTAA 1618
AGCTCAGTAA CTAAATAGTT TTTCAATCTT ATGCTTTAAT AAAAACGGGA TGATATTCTT 1678
ATGTAGGTTA AACTTATTAA AAATAAGTTT TGCAATCTGC TTGACTAGTT TATGATCAAC 1738
CTGGTTGATA GTTAATTTCT TAAGCATAAG AAGATTTTAA AATATTTAAA AAAACTATTG 1798
CTGATATGTT AAAATAGTTA AGGTATAAAA ATAATAAATT AAATATGGCT CGTAGACATG 1858
ATCTAACCGG GCTTGGTCCT TTAGCAGGAA ATAATCGTTC TCATGCTTTA AACATTACCA 1918
AGCGTCGTTG AAACTTAAAC CTACAAA 1945
(2) Information for SEQ ID NO. 16
   (i) Sequence characteristics:
      (A) Length of sequence: 1935
      (B) Type of sequence: amino acid
      (D) Topology: linear
      (E) Kind of sequence: DNA
   (xi) Indication of sequence: SEQ ID NO: 16 
TTTATTTTTA TTTTTGGTAA ATCTTTTAAA ATATAAATAT ATTTTAATAT TCT ATG 56
Met

AAT AAA AAA AGA ATC ATC TTA AAG ACT ATT AGC TTG TTA GGT ACA ACA 104
Asn Lys Lys Arg Ile Ile Leu Lys Thr Ile Ser Leu Leu Gly Thr Thr
            5                   10                  15
TCC TTT CTT AGT ATT GGG ATT TCT AGC TGT ATG TCT ATT ACT AAA AAA 152
Ser Phe Leu Ser Ile Gly Ile Ser Ser Cys Met Ser Ile Thr Lys Lys
        20                  25                  30
GAT GCA AAC CCA AAT AAT GGC CAA ACC CAA TTA GAA GCA GCG CGA ATG 200
Asp Ala Asn Pro Asn Asn Gly Gln Thr Gln Leu Glu Ala Ala Arg Met
    35                  40                  45
GAG TTA ACA GAT CTA ATC AAT GCT AAA GCG ATG ACA TTA GCT TCA CTA 248
Glu Leu Thr Asp Leu Ile Asn Ala Lys Ala Met Thr Leu Ala Ser Leu
50                  55                  60                  65
CAA GAC TAT GCC AAG ATT GAA GCT AGT TTA TCA TCT GCT TAT AGT GAA 296
Gln Asp Tyr Ala Lys Ile Glu Ala Ser Leu Ser Ser Ala Tyr Ser Glu
                70                  75                  80
GCT GAA ACA GTT AAC AAT AAC CTT AAT GCA ACA TTA GAA CAA CTA AAA 344
Ala Glu Thr Val Asn Asn Asn Leu Asn Ala Thr Leu Glu Gln Leu Lys
            85                  90                  95
ATG GCT AAA ACT AAT TTA GAA TCA GCC ATC AAC CAA GCT AAT ACG GAT 392
Met Ala Lys Thr Asn Leu Glu Ser Ala Ile Asn Gln Ala Asn Thr Asp
        100                 105                 110
AAA ACG ACT TTT GAT AAT GAA CAC CCA AAT TTA GTT GAA GCA TAC AAA 440
Lys Thr Thr Phe Asp Asn Glu His Pro Asn Leu Val Glu Ala Tyr Lys
    115                 120                 125
GCA CTA AAA ACC ACT TTA GAA CAA CGT GCT ACT AAC CTT GAA GGT TTG 488
 
Ala Leu Lys Thr Thr Leu Glu Gln Arg Ala Thr Asn Leu Glu Gly Leu
130                 135                 140                 145
TCA TCA ACT GCT TAT AAT CAA ATT CGC AAT AAT TTA GTG GAT CTA TAC 536
Ser Ser Thr Ala Tyr Asn Gln Ile Arg Asn Asn Leu Val Asp Leu Tyr
                150                 155                 160
AAT AAA GCT AGT AGT TTA ATA ACT AAA ACA CTA GAT CCA CTA AAT GGG 584
Asn Lys Ala Ser Ser Leu Ile Thr Lys Thr Leu Asp Pro Leu Asn Gly
            165                 170                 175
GGA ACG CTT TTA GAT TCT AAT GAG ATT ACT ACA GCT AAT AAG AAT ATT 632
Gly Thr Leu Leu Asp Ser Asn Glu Ile Thr Thr Ala Asn Lys Asn Ile
        180                 185                 190
AAT AAT ACG TTA TCA ACT ATT AAT GAA CAA AAG ACT AAT GCT GAT GCA 680
Asn Asn Thr Leu Ser Thr Ile Asn Glu Gln Lys Thr Asn Ala Asp Ala
    195                 200                 205
TTA GCT AAT AGT TTT ATT AAA GAA GTG ATT CAA AAT AAT AAA CAA AGT 728
Leu Ala Asn Ser Phe Ile Lys Glu Val Ile Gln Asn Asn Lys Gln Ser
210                 215                 220                 225
TTT GTA GGA ATG TTT ACA AAC ACT AAT GTT CAA CCT TCA AAC TAT AGT 776
Phe Val Gly Met Phe Thr Asn Thr Asn Val Gln Pro Ser Asn Tyr Ser
                230                 235                 240
TTT GTT GCT TTT AGT GCT GAT GTA ACA CCT GTT AAT TAT AAA TAT GCA 824
Phe Val Ala Phe Ser Ala Asp Val Thr Pro Val Asn Tyr Lys Tyr Ala
            245                 250                 255
AGA AGA ACG GTT TGA AAT GGT GAT GAA CCT TCA AGT AGA ATT CTT GCA 872
Arg Arg Thr Val Trp Asn Gly Asp Glu Pro Ser Ser Arg Ile Leu Ala
        260                 265                 270
AAC ACC AAT AGT ATT ACT GAT GTT TCA TGA ATT TAT AGT TTA TCT GGA 920
Asn Thr Asn Ser Ile Thr Asp Val Ser Trp Ile Tyr Ser Leu Ser Gly
    275                 280                 285
ACA AAC ACG AAA TAC CAA TTT AGT TTT AGC AAC TAC GGT CCA TCA ACT 968
 
Thr Asn Thr Lys Tyr Gln Phe Ser Phe Ser Asn Tyr Gly Pro Ser Thr
290                 295                 300                 305
GGT TAT TTA TAT TTC CCT TAT AAG TTG GTT AAA GCG GCT GAT GCT AGT     1016
Gly Tyr Leu Tyr Phe Pro Tyr Lys Leu Val Lys Ala Ala Asp Ala Ser
                310                 315                 320
AAT GTT GGA TTA CAA TAC AAA CTA AAT AAT GGA AAT GTT CAA CCA GTT     1064
Asn Val Gly Leu Gln Tyr Lys Leu Asn Asn Gly Asn Val Gln Pro Val
            325                 330                 335
GAG TTT GCC ACT TCA ACT AGC GCA AAT AAT ACT ACA GCT AAT CCA ACT     1112
Glu Phe Ala Thr Ser Thr Ser Ala Asn Asn Thr Thr Ala Asn Pro Thr
        340                 345                 350
CCA GCA GTT GAT GAG ATT AAA GTT GCT AAA ATC GTT TTA TCA GGT TTA     1160
Pro Ala Val Asp Glu Ile Lys Val Ala Lys Ile Val Leu Ser Gly Leu
    355                 360                 365
AGA TTT GGC CAA AAC ACA ATC GAA TTA AGT GTT CCA ACG GGT GAA AGA     1208
Arg Phe Gly Gln Asn Thr Ile Glu Leu Ser Val Pro Thr Gly Glu Arg
370                 375                 380                 385
AAT ATG AAT AAA GTT GCC CCA ATG ATT GGT AAT ATG TAT ATT ACT TCA     1256
Asn Met Asn Lys Val Ala Pro Met Ile Gly Asn Met Tyr Ile Thr Ser
                390                 395                 400
TCT AAT GCT GAA GCA AAT AAA AAG CAA ATT TAC GAT AGT ATT TTT GGA     1304
Ser Asn Ala Glu Ala Asn Lys Lys Gln Ile Tyr Asp Ser Ile Phe Gly
            405                 410                 415
AAC ACT TCA TCA CAA ACT GCT AGC CAA ACA TCT GTT AGT GTT GAT CTA     1352
Asn Thr Ser Ser Gln Thr Ala Ser Gln Thr Ser Val Ser Val Asp Leu
        420                 425                 430
TTA AAA GGA TAT AGT CTT GCA ACT AGT TCA AGA ACA TAT ATT CGT CAA     1400
Leu Lys Gly Tyr Ser Leu Ala Thr Ser Ser Arg Thr Tyr Ile Arg Gln
    435                 440                 445
TTT ACT GGT TTA ACA GAT AAT GGC GTA CAA ACC TCT GAC CCA GTT TAT     1448
 
Phe Thr Gly Leu Thr Asp Asn Gly Val Gln Thr Ser Asp Pro Val Tyr
450                 455                 460                 465
TTA ATT GGT TTG ATT GGT GGT CGT CAG GAT CGT ACA GTT GCA ACT GGT 1496
Leu Ile Gly Leu Ile Gly Gly Arg Gln Asp Arg Thr Val Ala Thr Gly
                470                 475                 480
ACA ACG AAT ATT CAA AAT TCT CCT GAT GTA GAT AAT GAT AAT AGA ACA 1544
Thr Thr Asn Ile Gln Asn Ser Pro Asp Val Asp Asn Asp Asn Arg Thr
            485                 490                 495
TTC ACA ATA TAT GTA AAT GCA CCA ATA AAC GGG AAC TAT CAC ATA AGT 1592
Phe Thr Ile Tyr Val Asn Ala Pro Ile Asn Gly Asn Tyr His Ile Ser
        500                 505                 510
GGT GCG TAT TTA CAA GGA ACC CGT ACA GCA AGA AGT CTG AAA TTC TCA 1640
Gly Ala Tyr Leu Gln Gly Thr Arg Thr Ala Arg Ser Leu Lys Phe Ser
    515                 520                 525
TCC GGT ACA AGT GGC AGT AAT AAT GAA GTT ACA GTC CTT GGT TTA GAA 1688
Ser Gly Thr Ser Gly Ser Asn Asn Glu Val Thr Val Leu Gly Leu Glu
530                 535                 540                 545
CAA AGG GAT TGA ACA ATA TTA GGT CAC TTT GAT ACA AAG ATG GAT GGT 1736
Gln Arg Asp Trp Thr Ile Leu Gly His Phe Asp Thr Lys Met Asp Gly
                550                 555                 560
ACT ACT ACT ATT TCA TGA ACA AAT ACA GCA AGC AAA AGA ACT CTA ACC 1784
Thr Thr Thr Ile Ser Trp Thr Asn Thr Ala Ser Lys Arg Thr Leu Thr
            565                 570                 575
TTA AAT AAA GGT CTA AAT AAA ATT ATT GTA AGT GGA GGA ACT CAA GAT 1832
Leu Asn Lys Gly Leu Asn Lys Ile Ile Val Ser Gly Gly Thr Gln Asp
        580                 585                 590
AAC ACA AAT GCT CCA TTT ATC GGT AAC TTA ACA TTT ACT CTC CAT CTA 1880
Asn Thr Asn Ala Pro Phe Ile Gly Asn Leu Thr Phe Thr Leu His Leu
    595                 600                 605
ACG TAGAAACTTC TATTGCAAGC TCTCAATCTC CACAACCAGT TAAAAATAGA TG 1935
Thr
610
(2) Information for SEQ ID NO. 17
   (i) Sequence characteristics:
      (A) Length of sequence: 32
      (B) Type of sequence: nucleic acid
      (C) Number of strand: single
      (D) Topology: linear
      (E) Kind of sequence: other nucleic acid, synthetic DNA
   (xi) Indication of sequence:
      TACGTTCTTCCTGGCAAACCTTACCACTACTT 32
(2) Information for SEQ ID NO. 18
   (i) Sequence characteristics:
      (A) Length of sequence: 21
      (B) Type of sequence: nucleic acid
      (C) Number of strand: single
      (D) Topology: linear
      (E) Kind of sequence: other nucleic acid, synthetic DNA
   (xi) Indication of sequence:
      CTACAAAGAACCTAAATATCA 21
(2) Information for SEQ ID NO. 19
   (i) Sequence characteristics:
      (A) Length of sequence: 24
      (B) Type of sequence: nucleic acid
      (C) Number of strand: single
      (D) Topology: linear
      (E) Kind of sequence: other nucleic acid, synthetic DNA
   (xi) Indication of sequence:
      TATAGAATTAAATTTTACTTATTC 24
(2) Information for SEQ ID NO. 20
   (i) Sequence characteristics:
      (A) Length of sequence: 97
      (B) Type of sequence: nucleic acid
      (C) Number of strand: single
      (D) Topology: linear
      (E) Kind of sequence: other nucleic acid, synthetic DNA
   (xi) Indication of sequence: 
AGCTTTTTTT TTTTTTTTTT TTTGGCATAT AAATAATAAA TACAATAATT AATTACGCGT 60

AAAAATTGAA AAACTATTCT AATTTATTGC ACTCGTC 97
(2) Information for SEQ ID NO. 21
   (i) Sequence characteristics:
      (A) Length of sequence: 93
      (B) Type of sequence: nucleic acid
      (C) Number of strand: single
      (D) Topology: linear
      (E) Kind of sequence: other nucleic acid, synthetic DNA
   (xi) Indication of sequence: 
AAAAAAAAAA AAAAAAAAAC CGTATATTTA TTATTTATGT TATTAATTAA TGCGCATTTT 60

TAACTTTTTG ATAAGATTAA ATAACGTGAG CAG 93
(2) Information for SEQ ID NO. 22
   (i) Sequence characteristics:
      (A) Length of sequence: 95
      (B) Type of sequence: nucleic acid
      (C) Number of strand: single
      (D) Topology: linear
      (E) Kind of sequence: other nucleic acid, synthetic DNA
   (xi) Indication of sequence: 
AGCTTTTTTT TTTTTTTTTT TTTGGCATAT AAATAATAAA TACAATAATT AATTACGCGT 60

AAAAATTGAA AAACTATTCT AATTTATTGC ACTCG 95
(2) Information for SEQ ID NO. 23
   (i) Sequence characteristics:
      (A) Length of sequence: 96
      (B) Type of sequence: nucleic acid
      (C) Number of strand: single
      (D) Topology: linear
      (E) Kind of sequence: other nucleic acid, synthetic DNA
   (xi) Indication of sequence: 
AAAAAAAAAA AAAAAAAAAA CCGTATATTT ATTATTTATG TTATTAATTA ATGCGCATTT 60

TTAACTTTTT GATAAGATTA AATAACGTGA GCCTAG 96
(2) Information for SEQ ID NO. 24
   (i) Sequence characteristics:
      (A) Length of sequence: 11
      (B) Type of sequence: nucleic acid
      (C) Number of strand: single
      (D) Topology: linear
      (E) Kind of sequence: other nucleic acid, synthetic DNA
   (xi) Indication of sequence:
      GATCCAGCATG 11
(2) Information for SEQ ID NO. 25
   (i) Sequence characteristics:
      (A) Length of sequence: 10
      (B) Type of sequence: nucleic acid
      (C) Number of strand: single
      (D) Topology: linear
      (E) Kind of sequence: other nucleic acid, synthetic DNA
   (xi) Indication of sequence:
      GTCGTACCTG 10
(2) Information for SEQ ID NO. 26
   (i) Sequence characteristics:
      (A) Length of sequence: 21
      (B) Type of sequence: nucleic acid
      (C) Number of strand: single
      (D) Topology: linear
      (E) Kind of sequence: other nucleic acid, synthetic DNA
   (xi) Indication of sequence:
      GGGATTTCGAATTCTATGTCT 21
(2) Information for SEQ ID NO. 27
   (i) Sequence characteristics:
      (A) Length of sequence: 2346
      (B) Type of sequence: amino acid
      (C) Number of strand: single
      (D) Topology: linear
      (E) Kind of sequence: DNA
   (xi) Indication of sequence: SEQ ID NO: 2.7 
AAAAACATCA GATTGTTAAT CTGATATCTT TGCTTAAAAA AACACAAAAT CTTCTAACAA 60
AATCCTAAAT AAATAAGCCG TTAAATTAAC TAAAAAATTA AAAAAATGGT TTTTCTTATC 120
AACCAAAATT CTCTAGTAAT AAACGCTTAT TTATTTTTAT TTTTAGTCAT CTTTTAAGAT 180
ATAAATATAT CTTAATATTC T ATG AAT AAG AAA AGA ATC ATC TTA AAG ACT 231
Met Asn Lys Lys Arg Ile Ile Leu Lys Thr
                5                   10
ATT AGT TTG TTA GGT ACA ACA TCC TTT CTT AGC ATT GGG ATT TCT AGC 279
Ile Ser Leu Leu Gly Thr Thr Ser Phe Leu Ser Ile Gly Ile Ser Ser
                15                  20                  25
TGT ATG TCT ATT ACT AAA AAA GAC GCA AAC CCA AAT AAT GGC CAA ACC 327
Cys Met Ser Ile Thr Lys Lys Asp Ala Asn Pro Asn Asn Gly Gln Thr
            30                  35                  40
CAA TTA CAA GCA GCG CGA ATG GAG TTA ACT GAT CTA ATC AAT GCT AAA 375
Gln Leu Gln Ala Ala Arg Met Glu Leu Thr Asp Leu Ile Asn Ala Lys
        45                  50                  55
GCA AGG ACA TTA GCT TCA CTA CAA GAC TAT GCT AAG ATT GAA GCT AGT 423
Ala Arg Thr Leu Ala Ser Leu Gln Asp Tyr Ala Lys Ile Glu Ala Ser
    60                  65                  70
TTA TCA TCT GCT TAT AGT GAA GCT GAA ACA GTT AAC AAT AAC CTT AAT 471
Leu Ser Ser Ala Tyr Ser Glu Ala Glu Thr Val Asn Asn Asn Leu Asn
75                  80                  85                  90
GCA ACA CTA GAA CAA CTA AAA ATG GCT AAA ACT AAT TTA GAA TCA GCC 519
Ala Thr Leu Glu Gln Leu Lys Met Ala Lys Thr Asn Leu Glu Ser Ala
                95                  100                 105
ATC AAC CAA GCT AAT ACG GAT AAA ACG ACT TTT GAT AAT GAA CAT CCA 567
Ile Asn Gln Ala Asn Thr Asp Lys Thr Thr Phe Asp Asn Glu His Pro
            110                 115                 120
AAT TTA GTT GAA GCA TAC AAA GCA CTA AAA ACC ACT TTA GAA CAA CGT 615
 
Asn Leu Val Glu Ala Tyr Lys Ala Leu Lys Thr Thr Leu Glu Gln Arg
        125                 130                 135
GCT ACT AAC CTT GAA GGT TTA GCT TCA ACT GCT TAT AAT CAG ATT CGT 663
Ala Thr Asn Leu Glu Gly Leu Ala Ser Thr Ala Tyr Asn Gln Ile Arg
    140                 145                 150
AAT AAT TTA GTG GAT CTA TAC AAT AAT GCT AGT AGT TTA ATA ACT AAA 711
Asn Asn Leu Val Asp Leu Tyr Asn Asn Ala Ser Ser Leu Ile Thr Lys
155                 160                 165                 170
ACA CTA GAT CCA CTA AAT GGG GGA ATG CTT TTA GAT TCT AAT GAG ATT 759
Thr Leu Asp Pro Leu Asn Gly Gly Met Leu Leu Asp Ser Asn Glu Ile
                175                 180                 185
ACT ACA GTT AAT CGG AAT ATT AAT AAT ACG TTA TCA ACT ATT AAT GAA 807
Thr Thr Val Asn Arg Asn Ile Asn Asn Thr Leu Ser Thr Ile Asn Glu
            190                 195                 200
CAA AAG ACT AAT GCT GAT GCA TTA TCT AAT AGT TTT ATT AAA AAA GTG 855
Gln Lys Thr Asn Ala Asp Ala Leu Ser Asn Ser Phe Ile Lys Lys Val
        205                 210                 215
ATT CAA AAT AAT GAA CAA AGT TTT GTA GGG ACT TTT ACA AAC GCT AAT 903
Ile Gln Asn Asn Glu Gln Ser Phe Val Gly Thr Phe Thr Asn Ala Asn
    220                 225                 230
GTT CAA CCT TCA AAC TAC AGT TTT GTT GCT TTT AGT GCT GAT GTA ACA 951
Val Gln Pro Ser Asn Tyr Ser Phe Val Ala Phe Ser Ala Asp Val Thr
235                 240                 245                 250
CCC GTC AAT TAT AAA TAT GCA AGA AGG ACC GTT TGG AAT GGT GAT GAA 999
Pro Val Asn Tyr Lys Tyr Ala Arg Arg Thr Val Trp Asn Gly Asp Glu
                255                 260                 265
CCT TCA AGT AGA ATT CTT GCA AAC ACG AAT AGT ATC ACA GAT GTT TCT 1047
Pro Ser Ser Arg Ile Leu Ala Asn Thr Asn Ser Ile Thr Asp Val Ser
            270                 275                 280
TGG ATT TAT AGT TTA GCT GGA ACA AAC ACG AAG TAC CAA TTT AGT TTT 1095
 
Trp Ile Tyr Ser Leu Ala Gly Thr Asn Thr Lys Tyr Gln Phe Ser Phe
        285                 290                 295
AGC AAC TAT GGT CCA TCA ACT GGT TAT TTA TAT TTC CCT TAT AAG TTG 1143
Ser Asn Tyr Gly Pro Ser Thr Gly Tyr Leu Tyr Phe Pro Tyr Lys Leu
    300                 305                 310
GTT AAA GCA GCT GAT GCT AAT AAC GTT GGA TTA CAA TAC AAA TTA AAT 1191
Val Lys Ala Ala Asp Ala Asn Asn Val Gly Leu Gln Tyr Lys Leu Asn
315                 320                 325                 330
AAT GGA AAT GTT CAA CAA GTT GAG TTT GCC ACT TCA ACT AGT GCA AAT 1239
Asn Gly Asn Val Gln Gln Val Glu Phe Ala Thr Ser Thr Ser Ala Asn
                335                 340                 345
AAT ACT ACA GCT AAT CCA ACT CCA GCA GTT GAT GAG ATT AAA GTT GCT 1287
Asn Thr Thr Ala Asn Pro Thr Pro Ala Val Asp Glu Ile Lys Val Ala
            350                 355                 360
AAA ATC GTT TTA TCA GGT TTA AGA TTT GGC CAA AAC ACA ATC GAA TTA 1335
Lys Ile Val Leu Ser Gly Leu Arg Phe Gly Gln Asn Thr Ile Glu Leu
        365                 370                 375
AGT GTT CCA ACG GGT GAA GGA AAT ATG AAT AAA GTT GCG CCA ATG ATT 1383
Ser Val Pro Thr Gly Glu Gly Asn Met Asn Lys Val Ala Pro Met Ile
    380                 385                 390
GGC AAC ATT TAT CTT AGC TCA AAT GAA AAT AAT GCT GAT AAG ATC TAC 1431
Gly Asn Ile Tyr Leu Ser Ser Asn Glu Asn Asn Ala Asp Lys Ile Tyr
395                 400                 405                 410
AAT GAT ATC TTT GGT AAC ACA ATC AAC CAA CAG AAT AAT GCT ATT TCT 1479
Asn Asp Ile Phe Gly Asn Thr Ile Asn Gln Gln Asn Asn Ala Ile Ser
                415                 420                 425
GTA ATG GTT AAT ATG GTT GAG GGA TAT AAT TTA GCT AGT AGT TAT TCT 1527
Val Met Val Asn Met Val Glu Gly Tyr Asn Leu Ala Ser Ser Tyr Ser
            430                 435                 440
CCA GCA TAT AAA CCA ATT AAT GTT TCC ACT GGT GGT GGT CAA ACT CAA 1575
 
Pro Ala Tyr Lys Pro Ile Asn Val Ser Thr Gly Gly Gly Gln Thr Gln
        445                 450                 455
CCA TAT TAT GTA ATT GGT TGA TTG GGC GCT AGT GAT CAG AAC CCT AGA 1623
Pro Tyr Tyr Val Ile Gly Trp Leu Gly Ala Ser Asp Gln Asn Pro Arg
    460                 465                 470
AAC GCT GTG GGA ACC AAC ATG AAC GTA CAA AGA GTT CCA GCA ACA AAT 1671
Asn Ala Val Gly Thr Asn Met Asn Val Gln Arg Val Pro Ala Thr Asn
475                 480                 485                 490
AGC AAC CAA GGC GGA TAT GCT AGA TAT GTC TCT TTT TAT GTT AAT GCT 1719
Ser Asn Gln Gly Gly Tyr Ala Arg Tyr Val Ser Phe Tyr Val Asn Ala
                495                 500                 505
CCA CAA GCT GGT TCA TAT TAT ATT AGT GGT AAC TAT AAT AGT TTA ACA 1767
Pro Gln Ala Gly Ser Tyr Tyr Ile Ser Gly Asn Tyr Asn Ser Leu Thr
            510                 515                 520
GCT AGA GGT CTA GCT GTG TCT ACT GAG AAA ACA TTT ACA ACC AAT GTG 1815
Ala Arg Gly Leu Ala Val Ser Thr Glu Lys Thr Phe Thr Thr Asn Val
        525                 530                 535
ATC AAG ATC ACT CAC TTA CAA GTA ATT AAT GCC ACT AAT AGA ATC TTA 1863
Ile Lys Ile Thr His Leu Gln Val Ile Asn Ala Thr Asn Arg Ile Leu
    540                 545                 550
ACC TTT GAT ACT AAA ACA AAA AGA GGA ACT GAT AGT AAT AAC GGT AAT 1911
Thr Phe Asp Thr Lys Thr Lys Arg Gly Thr Asp Ser Asn Asn Gly Asn
555                 560                 565                 570
ATT ACA TTA GAA GCA AAC AAA GAC ACA ATA ACA TTA ACT AAG GGT TGA 1959
Ile Thr Leu Glu Ala Asn Lys Asp Thr Ile Thr Leu Thr Lys Gly Trp
                575                 580                 585
AAC AAA CTT TAT GTT TCA GGT AAT AAT AAT GAC ACT GTA GGT ATT GGT 2007
Asn Lys Leu Tyr Val Ser Gly Asn Asn Asn Asp Ser Val Gly Ile Gly
            590                 595                 600
AAT CTT ACT TTT ACA TTA ATG CCA CCA CAA ACT AAT TCA TAATTAAGAT 2056
 
Asn Leu Thr Phe Thr Leu Met Pro Pro Gln Thr Asn Ser
        605                 610                 615
ATATTAAACA TACCCATTTA GATAATCTAA ATGGGTATCT TTTTTATTGA AAATGGCGCA 2116
TGATGAAATC AAAGTTAAGT TCACTAGTGC TTTGATAAAT TAGATCAGCT TTAGAANNAT 2176
CTTCACTACT GCCATCGGTA ATGACAACAG CTTTCATTTT GNCTGCTTCC ATCGCTTTCA 2236
ATCCTGAGAT CGCATCTTCA AACCCAATAN CTTNATCATT GCTGATATCT AAGTCTTCTN 2296
CAGCTTTAAG ATAGATATCA GCTNCTGGTT TACCTTGGTT AATCTCACTT 2346
(2) Information for SEQ ID NO. 28
   (i) Sequence characteristics:
      (A) Length of sequence: 17
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 28
      GTTTTCCCAGTCACGAC 17
(2) Information for SEQ ID NO. 29
   (i) Sequence characteristics:
      (A) Length of sequence: 27
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 29
      AACCAACCAACCCGATCGCTAGTCT 27
(2) Information for SEQ ID NO. 30
   (i) Sequence characteristics:
      (A) Length of sequence: 20
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 30
      TGATTGGGCGCTAGCGATCA 20
(2) Information for SEQ ID NO. 31
   (i) Sequence characteristics:
      (A) Length of sequence: 23
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 31
      TGCCAACCTTGTTCGAAATACAA 23
(2) Information for SEQ ID NO. 32
   (i) Sequence characteristics:
      (A) Length of sequence: 19
      (B) Type of sequence: nucleic acid
      (C) Number of strand: single
      (D) Topology: linear
      (E) Kind of sequence: other nucleic acid, DNA
   (xi) Indication of sequence: SEQ ID NO: 32
      TGAAACAAGCTTTATGTTT 19
(2) Information for SEQ ID NO. 33
   (i) Sequence characteristics:
      (A) Length of sequence: 17
      (B) Type of sequence: nucleic acid
      (C) Number of strand: single
      (D) Topology: linear
      (E) Kind of sequence: other nucleic acid, DNA
   (xi) Indication of sequence: SEQ ID NO: 33
      CAGTATCGACAAAGGAC 17
(2) Information for SEQ ID NO. 34
   (i) Sequence characteristics:
      (A) Length of sequence: 17
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 34
      CAGGAAACAGCTATGAC . 17
(2) Information for SEQ ID NO. 35
   (i) Sequence characteristics:
      (A) Length of sequence: 20
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 35
      GTTCTTCCTGGCAAACTTTA 20
(2) Information for SEQ ID NO. 36
   (i) Sequence characteristics:
      (A) Length of sequence: 20
      (B) Type of sequence: nucleic acid
      (C) Number of strand: single
      (D) Topology: linear
      (E) Kind of sequence: other nucleic acid, - synthetic DNA
   (xi) Indication of sequence: SEQ ID NO: 36
      AAGAAGGACCGTTTGGAATG 20
(2) Information for SEQ ID NO. 37
   (i) Sequence characteristics:
      (A) Length of sequence: 17
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 37
      GTTTTCCCAGTCACGAC 17
(2) Information for SEQ ID NO. 38
   (i) Sequence characteristics:
      (A) Length of sequence: 27
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 38
      CAAAGTACCTAAATATCGAATTCACCT 27
(2) Information for SEQ ID NO. 39
   (i) Sequence characteristics:
      (A) Length of sequence: 23
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 39
      ATAGCTTAAGTGGAACAAACACG 23
(2) Information for SEQ ID NO. 40
   (i) Sequence characteristics:
      (A) Length of sequence: 20
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 40
      GGAACCAGATCTTGTTTCCC 20
(2) Information for SEQ ID NO. 41
   (i) Sequence characteristics:
      (A) Length of sequence: 21
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 41
      GGTCTAGAACAAAGGGATTGGACA 21
(2) Information for SEQ ID NO. 42
   (i) Sequence characteristics:
      (A) Length of sequence: 20
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 42
      CTACCTACCATGGTGATGAT 20
(2) Information for SEQ ID NO. 43
   (i) Sequence characteristics:
      (A) Length of sequence: 27
      (B) Type of sequence: nucleic acid
      (C) Number of strand: single
      (D) Topology: linear
      (E) Kind of sequence: other nucleic acid, synthetic DNA
   (xi) Indication of sequence: SEQ ID NO: 43
      GATGGTACCACTACTATTTCATGGACA 27

## Claims

1. A substantially pure antigenic protein which is reactive with *Mycoplasma gallisepticum-*immunized sera or *Mycoplasma* gallisepticum-infected sera and which has the amino acid sequence shown by SEQ ID NO: 27.

2. An isolated DNA encoding an antigenic protein according to claim 1.

3. A fused protein comprising a polypeptide of *Mycoplasma gallisepticum* ligated at the N-terminus thereof with a signal membrane anchor of type II external membrane protein of a virus that infects poultry, wherein said polypeptide of *Mycoplasma gallisepticum* has the amino acid sequence shown by SEQ ID NO: 27.

4. A fused protein according to claim 3 wherein the signal membrane anchor is encoded by DNA comprising SEQ ID NO: 13.

5. A hybrid DNA encoding the fused protein according to claim 3 or 4.

6. A component vaccine comprising a protein according to claim 1, 3 or 4.

7. A recombinant Avipox virus comprising DNA encoding a polypeptide of *Mycoplasma gallisepticum,* wherein said polypeptide of *Mycoplasma gallisepticum* has the amino acid sequence of SEQ ID NO: 27.

8. The recombinant Avipox virus according to claim 7, wherein the polypeptide of *Mycoplasma gallisepticum* has at the N-terminus thereof, a signal membrane anchor of a type II external membrane protein from a virus that infects poultry.

9. A live vaccine for poultry *Mycoplasma gallisepticum* infection comprising a live recombinant Avipox virus according to claim 8.

10. A fused protein according to claim 3 or 4, wherein a hydrophilic sequence having between 10 and 50 amino acids is present at the carboxy terminal of the signal membrane anchor.

## Patentansprüche

1. Im Wesentlichen reines antigenes Protein, das mit *Mycoplasma gallisepticum*immunisierten Seren oder *Mycoplasma gallisepticum-infizierten* Seren reaktiv ist und das die in SEQ ID NO:27 dargestellte Aminosäuresequenz hat.

2. Isolierte DNA, die ein antigenes Protein nach Anspruch 1 codiert.

3. Fusionsprotein, umfassend ein Polypeptid von *Mycoplasma gallisepticum,* das an seinem N-Terminus mit einem Signalmembrananker eines äußere Membran-Proteins vom Typ II eines Virus, das Geflügel infiziert, verknüpft ist, wobei das Polypeptid von *Mycoplasma gallisepticum* die in SEQ ID NO:27 dargestellte Aminosäuresequenz hat.

4. Fusionsprotein nach Anspruch 3, wobei der Signalmembrananker durch eine DNA codiert wird, die SEQ ID NO:13 umfasst.

5. Hybrid-DNA, die das fusionierte Protein nach Anspruch 3 oder 4 codiert.

6. Komponenten-Impfstoff, umfassend ein Protein nach einem der Ansprüche 1, 3 oder 4.

7. Rekombinantes Avipox-Virus umfassend DNA, die ein Polypeptid von *Mycoplasma gallisepticum* codiert, wobei das Polypeptid von *Mycoplasma gallisepticum* die in SEQ ID NO:27 dargestellte Aminoäuresequenz hat.

8. Rekombinantes Avipox-Virus nach Anspruch 7, wobei das Polypeptid von *Mycoplasma gallisepticum* an seinem N-Terminus einen Signalmembrananker eines äußere Membran-Proteins vom Typ II eines Virus, das Geflügel infiziert, hat.

9. Lebendimpfstoff gegen eine *Mycoplasma gallisepticum-Infektion* in Geflügel, umfassend ein lebendes rekombinantes Avipox-Virus nach Anspruch 8.

10. Fusionsprotein nach Anspruch 3 oder 4, wobei eine hydrophile Sequenz, die zwischen 10 und 50 Aminosäuren hat, am Carboxyterminus des Signalmembranankers vorhanden ist.

## Revendications

1. Protéine antigénique sensiblement pure, qui peut réagir avec des sérums immunisés contre *Mycoplasma gallisepticum* ou des sérums infectés par *Mycoplasma gallisepticum* et qui présente la séquence d'acides aminés donnée en tant que Séquence N° 27.

2. ADN isolé, codant une protéine antigénique conforme à la revendication 1.

3. Protéine de fusion, comprenant un polypeptide de *Mycoplasma gallisepticum* qui porte, liée à son extrémité amino-terminale, une séquence signal-ancrage d'une protéine de membrane externe de type II d'un virus qui infecte les volailles, dans laquelle ledit polypeptide de *Mycoplasma gallisepticum* présente la séquence d'acides aminés donnée en tant que Séquence N° 27.

4. Protéine de fusion conforme à la revendication 3, dans laquelle la séquence signal-ancrage est codée par un ADN comprenant la Séquence N° 13.

5. ADN hybride codant une protéine de fusion conforme à la revendication 3 ou 4.

6. Vaccin à composant comprenant une protéine conforme à la revendication 1, 3 ou 4.

7. Virus de la variole aviaire recombinant, comprenant un ADN codant un polypeptide de *Mycoplasma gallisepticum,* dans lequel ce polypeptide de *Mycoplasma gallisepticum* présente la séquence d'acides aminés donnée en tant que Séquence N° 27.

8. Virus de la variole aviaire recombinant, conforme à la revendication 7, dans lequel le polypeptide de *Mycoplasma gallisepticum* porte, à son extrémité amino-terminale, une séquence signal-ancrage d'une protéine de membrane externe de type II d'un virus qui infecte les volailles.

9. Vaccin vivant, dirigé contre une infection par *Mycoplasma gallisepticum* chez des volailles, comprenant un virus de la variole aviaire recombinant vivant, conforme à la revendication 8.

10. Protéine de fusion conforme à la revendication 3 ou 4, dans laquelle se trouve, à l'extrémité carboxy-terminale de la séquence signal-ancrage, une séquence hydrophile comportant de 10 à 50 résidus d'acides aminés.
